# EUROPEAN PATENT APPLICATION

(11) **EP 3 673 891 A1**
(43) Date of publication of application: **01.07.2020**
(21) Application number: 18848059.4
(22) Date of filing: 09.08.2018
(51) Int. Cl.: A61J 3/00, G01B 11/24, G01N 21/85, G06T 1/00

(54) **MEDICINE INSPECTION SUPPORT DEVICE, IMAGE PROCESSING DEVICE, IMAGE PROCESSING METHOD, AND PROGRAM**

(30) Priority: 25.08.2017 JP 2017162545; 10.01.2018 JP 2018002006; 23.05.2018 WO PCT/JP2018/019857
(71) Applicant: FUJIFILM Toyama Chemical Co., Ltd., Chuo-ku Tokyo 1040031 (JP)
(72) Inventor: YOKOUCHI, Koji, Ashigarakami-gun Kanagawa 258-8538 (JP); TAKASHIMA, Masanobu, Ashigarakami-gun Kanagawa 258-8538 (JP)
(74) Representative: Hoffmann Eitle
(86) International application number: PCT/JP2018/029928
(87) International publication number: WO 2019/039302

(57) **Abstract**

A medicine inspection support device, an image processing device, an image processing method and a program are provided that appropriately recognize identification information irrespective of whether the identification information is an engraved mark or a printed character. The image processing device obtains a plurality of taken images of a second medicine with light illumination directions to the surface of the second medicine different from each other, generates a first enhanced image by performing a process of enhancing an engraved mark portion of the second medicine, generates a second enhanced image by performing a process of enhancing a printed character portion of the second medicine, and collates the image designated by the designation information designating one of the first enhanced image and the second enhanced image as an image to be used for collation with the master image to determine whether the first medicine is identical to the second medicine.

## Description

### BACKGROUND OF THE INVENTION

### 1. Field of the Invention

The present invention relates to a medicine inspection support device, an image processing device, an image processing method, and a program, and in particular, to a medicine inspection support device, an image processing device, an image processing method, and a program that identify the kind of a medicine from an image obtained by imaging the medicine.

### 2. Description of the Related Art

Medicines have information for identifying the medicines added thereon. The identification information is read to identify the kind of a medicine. Methods of adding the identification information include medicines having engraved marks and medicines having printed characters.

Japanese Patent Application Laid-Open No. 2015-232483 (hereinafter referred to as "PTL 1") describes a technique that uses photometric stereo to create a contour extraction image representing a height in grayscale (luminance) in an image, thereby inspecting a scar and a printed character. According to the technique of PTL 1, it is possible to achieve image inspection that is hardly affected by an inclination of a workpiece or illumination and is robust against an environment.

### Citation List

PTL 1: Japanese Patent Application Laid-Open No. 2015-232483

### SUMMARY OF THE INVENTION

Characters or the like printed on the surface of a medicine have no convexoconcave on the surface. Accordingly, in a case where a contour extraction image is generated as in the device described in PTL 1, printed information cannot be recognized. In addition, it is left unknown whether the identification information has been added to a collation target medicine by mark engraving or by character printing.

Accordingly, there is a problem in that it is unknown which image processing is preferable for a taken image.

The present invention has been made in view of such situations, and aims to provide a medicine inspection support device, an image processing device, an image processing method and a program that appropriately recognize identification information irrespective of whether the identification information is an engraved mark or a printed character.

To achieve the above object, an aspect of an image processing device includes: a master image obtaining unit configured to obtain a master image of a first medicine; a taken image obtaining unit configured to obtain a plurality of taken images of a second medicine with light emitting directions to a surface of the second medicine different from each other; a first image processing unit configured to perform a process of enhancing an engraved mark portion of the second medicine based on at least one taken image among the taken images, and generate a first enhanced image; a second image processing unit configured to perform a process of enhancing a printed character portion of the second medicine based on at least one taken image among the taken images, and generate a second enhanced image; a designation information obtaining unit configured to obtain designation information designating one of the first enhanced image and the second enhanced image, as an image to be used for collation; a determining unit configured to collate the image designated by the designation information against the master image, and determine whether the first medicine is identical to the second medicine or not.

According to this aspect, the master image of the first medicine is obtained, the taken images of the medicine with the light illumination directions to the surface of the second medicine different from each other are obtained, the first enhanced image is generated by performing the process of enhancing the engraved mark portion of the second medicine, the second enhanced image is generated by performing the process of enhancing the printed character portion of the second medicine, and the image designated by the designation information designating one of the first enhanced image and the second enhanced image as an image to be used for collation is collated against the master image to determine whether the first medicine is identical to the second medicine or not. Accordingly, the identification information can be appropriately recognized irrespective of whether the identification information is engraved marks or printed characters.

Preferably, the master image and the designation information are stored in association with each other. Accordingly, the designation information can be appropriately obtained.

Preferably, the image processing device further includes a medicine detecting unit configured to detect areas where the second medicine are imaged from each of the taken images, and the first image processing unit and the second image processing unit perform the processes for the detected areas. Accordingly, the areas to be processed can be limited, which can reduce the time required to the processes.

Preferably, the taken image obtaining unit obtains three or more taken images, and the first image processing unit generates the first enhanced image based on the three or more taken images. Accordingly, the engraved mark portion of the medicine can be appropriately enhanced.

Preferably, the first image processing unit obtains three-dimensional information on the surface of the second medicine by photometric stereo, and generates the first enhanced image. Accordingly, the engraved mark portion of the medicine can be appropriately enhanced.

Preferably, the second image processing unit performs at least one of a smoothing process, a sharpening process, and an edge detecting process, and generates the second enhanced image. Accordingly, the printed character portion of the medicine can be appropriately enhanced.

Preferably, the image processing device further includes a similarity calculating unit configured to calculate a similarity indicating a degree at which the image designated by the designation information is similar to the master image, and the determining unit determines whether the first medicine is identical to the second medicine or not based on the similarity. Accordingly, it can be appropriately determined whether the first medicine is identical to the second medicine or not.

Preferably, the similarity calculating unit calculates similarities between a plurality of images designated by the designation information and a plurality of master images, and the determining unit determines that the first medicine is identical to the second medicine, for a combination between the image designated by the designation information and a master image, with a highest similarity. Accordingly, it can be appropriately determined whether the first medicine is identical to the second medicine or not.

Preferably, the master image obtaining unit obtains the master image of the first medicine based on prescription data. Accordingly, the master image of the first medicine can be appropriately obtained. The prescription data includes information described on the prescription, information set or changed based on the information described on the prescription, and information input by a user.

To achieve the above object, an aspect of a medicine inspection support device includes: a master image obtaining unit configured to obtain a master image of a first medicine; an illuminating unit including a plurality of light sources and configured to irradiate a surface of a second medicine with light in a plurality of illumination directions different from each other; an imaging unit configured to image the second medicine; an imaging control unit configured to control the illuminating unit and the imaging unit, and obtain a plurality of taken images of the second medicine with light illumination directions to the second medicine different from each other; a first image processing unit configured to perform a process of enhancing an engraved mark portion of the second medicine based on at least one taken image among the taken images, and generate a first enhanced image; a second image processing unit configured to perform a process of enhancing a printed character portion of the second medicine based on at least one taken image among the taken images, and generate a second enhanced image; a designation information obtaining unit configured to obtain designation information designating one of the first enhanced image and the second enhanced image as an image to be used for collation; and a determining unit configured to collate the image designated by the designation information against the master image, and determine whether the first medicine is identical to the second medicine or not.

According to this aspect, the master image of the first medicine is obtained, the taken images of the second medicine with light illumination directions to the surface of the second medicine different from each other are obtained, the first enhanced image is generated by performing the process of enhancing the engraved mark portion of the second medicine, the second enhanced image is generated by performing the process of enhancing the printed character portion of the second medicine, and the image designated by the designation information designating one of the first enhanced image and the second enhanced image as an image to be used for collation is collated against the master image to determine whether the first medicine is identical to the second medicine or not. Accordingly, the identification information can be appropriately recognized irrespective of whether the identification information is the engraved mark or the printed character.

Preferably, a master image storage unit configured to store the master image of the first medicine, is provided, and the designation information is stored in the master image storage unit in association with the master image. Accordingly, the designation information can be appropriately obtained.

Preferably, the imaging control unit obtains the taken images of the second medicine irradiated with light by two or more light sources among the light sources of the illuminating unit. Accordingly, the printed character portion of the medicine can be appropriately enhanced.

Preferably, the illuminating unit irradiates a front side and a rear side of the second medicine with light, and the imaging unit images the front side and the rear side of the second medicine. Accordingly, an appropriate taken image can be obtained irrespective of the posture of the medicine.

Preferably, the illuminating unit includes a first light source configured to emit light in a first direction, a second light source configured to emit light in a second direction, a third light source configured to emit light in a third direction, and a fourth light source configured to emit light in a fourth direction, and the second direction is a direction opposite to the first direction in plan view of the surface, the third direction is a direction orthogonal to the first direction in plan view of the surface, and the fourth direction is a direction opposite to the third direction in plan view of the surface. Accordingly, a plurality of taken images of the medicine where light illumination directions to the medicine are different from each other, can be appropriately obtained.

To achieve the above object, an aspect of an image processing method includes: a master image obtaining step of obtaining a master image of a first medicine; a taken image obtaining step of obtaining a plurality of taken images of a medicine, with light emitting directions to a surface of the second medicine different from each other; a first image processing step of performing a process of enhancing an engraved mark portion of the second medicine based on at least one taken image among the taken images, and generating a first enhanced image; a second image processing step of performing a process of enhancing a printed character portion of the second medicine based on at least one taken image among the taken images, and generating a second enhanced image; a designation information obtaining step of obtaining designation information designating one of the first enhanced image and the second enhanced image, as an image to be used for collation; and a determining step of collating the image designated by the designation information against the master image, and determining whether the first medicine is identical to the second medicine or not.

According to this aspect, the master image of the first medicine is obtained, the plurality of taken images of the medicine with light illumination directions to the surface of the second medicine different from each other are obtained, the first enhanced image is generated by performing the process of enhancing the engraved mark portion of the second medicine, the second enhanced image is generated by performing the process of enhancing the printed character portion of the second medicine, and the image designated by the designation information designating one of the first enhanced image and the second enhanced image as an image to be used for collation is collated against the master image to determine whether the first medicine is identical to the second medicine or not. Accordingly, the identification information can be appropriately recognized irrespective of whether the identification information is the engraved mark or the printed character.

To achieve the above object, an aspect of a program causes a computer to execute: a master image obtaining function of obtaining a master image of a first medicine; a taken image obtaining function of obtaining a plurality of taken images of a second medicine, with light emitting directions to a surface of the second medicine different from each other; a first image processing function of performing a process of enhancing an engraved mark portion of the second medicine based on at least one taken image among the taken images, and generating a first enhanced image; a second image processing function of performing a process of enhancing a printed character portion of the second medicine based on at least one taken image among the taken images, and generating a second enhanced image; a designation information obtaining function of obtaining designation information designating one of the first enhanced image and the second enhanced image, as an image to be used for collation; and a determining function of collating the image designated by the designation information against the master image, and determining whether the first medicine is identical to the second medicine or not.

According to this aspect, the master image of the first medicine is obtained, the taken images of the second medicine with light illumination directions to the surface of the second medicine different from each other are obtained, the first enhanced image is generated by performing the process of enhancing the engraved mark portion of the second medicine, the second enhanced image is generated by performing the process of enhancing the printed character portion of the second medicine, and the image designated by the designation information designating one of the first enhanced image and the second enhanced image as an image to be used for collation is collated against the master image to determine whether the first medicine is identical to the second medicine or not. Accordingly, the identification information can be appropriately recognized irrespective of whether the identification information is the engraved mark or the printed character.

To achieve the above object, an aspect of a medicine inspection support device is a medicine inspection support device inspecting a medicine, including: a medicine discriminating unit configured to collate a medicine master image from a medicine database including medicine master images with a taken image obtained by imaging a medicine to be inspected, and determine which medicine the medicine present in the taken image is; and a list creating unit configured to create a list table of medicines to be inspected, the list table displaying the medicine master image and a medicine area image determined to correspond to each medicine in the taken image, with positions of the medicine master image and the medicine area image being aligned to each other, and an engraved mark portion or a printed character portion being enhanced.

According to this aspect, the medicine master image of the medicine, and the medicine area image in the taken image are displayed such that the positions are aligned to each other, and the engraved mark portion or the printed character portion is enhanced. Irrespective of whether the identification information is the engraved mark or the printed character, the user can be allowed to recognize the identification information appropriately.

According to the present invention, irrespective of whether the identification information is an engraved mark or a printed character, the identification information can be appropriately recognized.

### BRIEF DESCRIPTION OF DRAWINGS

Figure 1 shows a configuration of a medicine inspection support device.
Figure 2 is a side view showing situations where images of a divided package are obtained.
Figure 3 is a top view showing situations where the images of the divided package are obtained.
Figure 4 shows an internal configuration of a processing unit.
Figure 5 is a flowchart showing processes of a medicine inspection support method.
Figure 6 shows an example of image processing results.
Figure 7 shows an example of image processing results.
Figure 8 shows an example of image processing results.
Figure 9 shows an example of image processing results.
Figure 10 shows a configuration of a medicine inspection support device.
Figure 11 shows an internal configuration of a processing unit.
Figure 12 is a flowchart showing processes of a medicine inspection support method.
Figure 13 is a flowchart showing processes of a medicine inspection support method.
Figure 14 shows a configuration of a medicine inspection support device.
Figure 15 is a side view showing situations where images of a divided package are obtained.
Figure 16 is a top view showing situations where the images of the divided package are obtained.
Figure 17 is a flowchart showing processes of a medicine inspection support method.
Figure 18 shows a configuration of a medicine inspection support device.
Figure 19 is a flowchart showing processes of a medicine inspection support method.
Figure 20 is a flowchart showing processes of a medicine inspection support method.
Figure 21 shows a configuration of a medicine inspection support device.
Figure 22 shows an internal configuration of a processing unit.
Figure 23 is a flowchart showing processes of a medicine inspection support method.
Figure 24 shows a list table.
Figure 25 shows a list table in a comparative example.

### DESCRIPTION OF THE PREFERRED EMBODIMENTS

Hereinafter, referring to the accompanying drawings, preferred embodiments of the present invention are described in detail.

### <First embodiment

A medicine inspection support device according to a first embodiment is a device that supports an audit of a medicine by collating a master image with a taken image. The medicine inspection support device accurately performs collation irrespective of whether identification information added to the medicine has been added by mark engraving or by character printing.

Note that addition by mark engraving means that the identification information by formed by forming a groove that is a depressed (recessed) area on a surface of a medicine. The groove is not limited to what has been formed by scraping the surface. Alternatively, the groove may be what has been formed by pressing the surface. The engraved mark may include what does not have an identification function, such as of a cleavage line.

Addition by character printing means that the identification information is formed by adding edible ink or the like through contact or noncontact on a surface of a tablet. Here, addition by character printing has the same meaning as that of addition by printing.

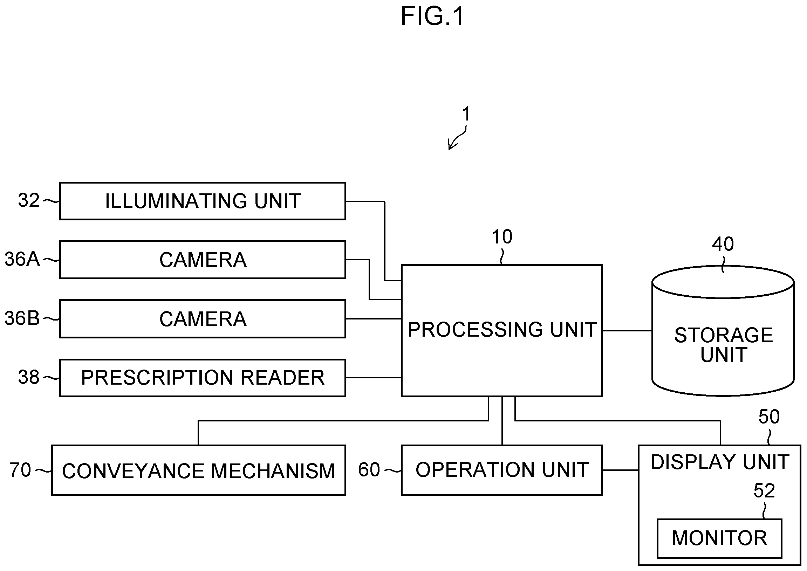

Figure 1 shows a configuration of a medicine inspection support device 1 according to the first embodiment. The medicine inspection support device 1 includes a processing unit 10, a storage unit 40, a display unit 50, an operation unit 60, and a conveyance mechanism 70. Furthermore, an illuminating unit 32, a camera 36A, a camera 36B, and a prescription reader 38 are connected to the processing unit 10.

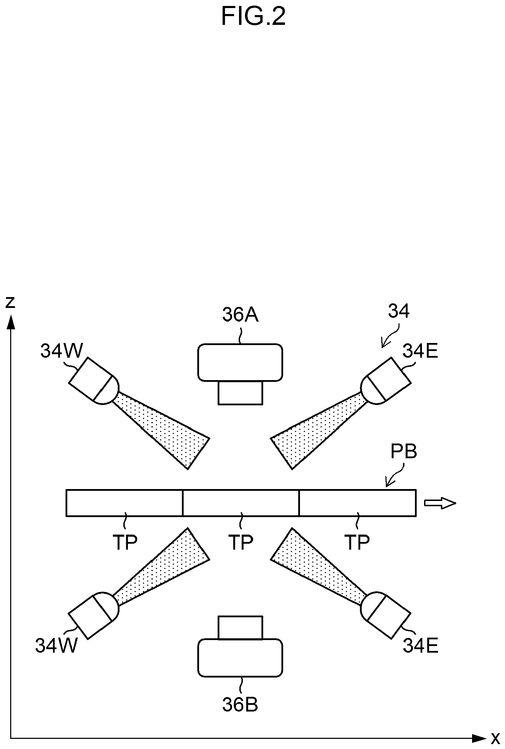

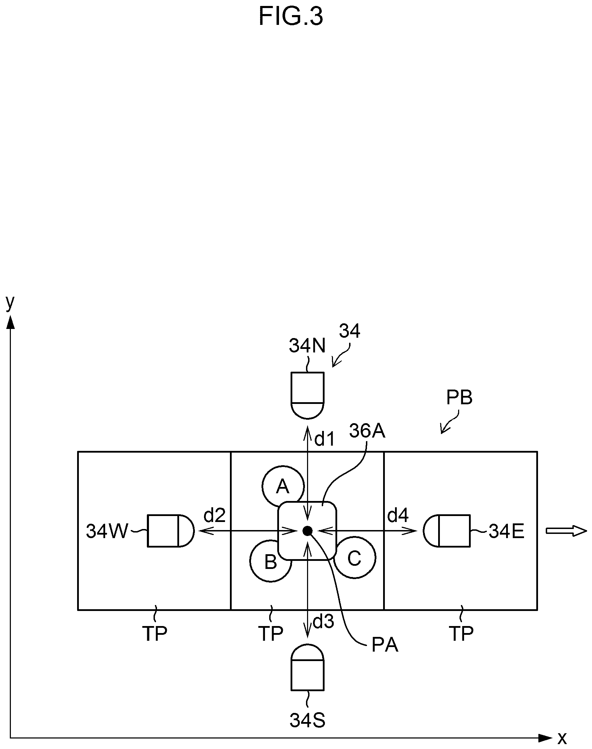

Figure 2 is a side view showing situations where the medicine inspection support device 1 obtains images of (medicines packaged in) a divided package. Figure 3 is a top view thereof.

The illuminating unit 32 includes a plurality of light sources 34 that each irradiate, with light, a strip medicine package PB including divided packages TP in series. In each of the divided packages TP, a dispensed medicine is packaged in a divided manner. As the light sources 34, four light sources 34N, 34S, 34E and 34W are arranged on each of an upward side (+z direction side in Figure 2) and a lower side (-z direction side in Figure 2) of the strip medicine package PB. Note that illustration of the light sources 34N and 34S on the upper side and the lower side of the strip medicine package PB is omitted in Figure 2, and illustration of the four light sources 34N, 34S, 34E and 34W on the lower side of the strip medicine package PB is omitted in Figure 3.

The light sources 34N (example of first light source), 34S (example of second light source), 34E (example of third light source) and 34W (example of fourth light source) above the strip medicine package PB emit light respectively in -y direction (example of first direction), +y direction (example of second direction), -x direction (example of third direction) and +x direction (example of fourth direction) in the xy-plan view of Figure 3. That is, the illumination direction of the light source 34S is the direction opposite to the illumination direction of the light source 34N in xy-plan view, the illumination direction of the light source 34E is the direction orthogonal to the illumination direction of the light source 34N in xy-plan view, and the illumination direction of the light source 34W is the direction opposite to the illumination direction of the light source 34E in xy-plan view.

The light sources 34N, 34S, 34E and 34W below the strip medicine package PB are also arranged in a similar manner. Accordingly, the illuminating unit 32 irradiates the front side and the rear side of (the medicine packaged in) the divided package TP with light.

Each of the cameras 36A and 36B (example of imaging unit) includes a digital camera. As shown in Figure 2, the camera 36A is arranged above the strip medicine package PB, and the camera 36B is arranged below the strip medicine package PB. The cameras 36A and 36B image the front side and the rear side of (the medicine packaged in) the divided package TP.

The divided packages TP (strip medicine package PB) are conveyed by the conveyance mechanism 70 in +x direction (the longitudinal direction of the strip medicine package PB) in Figure 3. During imaging, the upper side of the divided package TP is illuminated in four directions by the light sources 34N, 34S, 34E and 34W above the divided package TP, and the lower side of the divided package TP is illuminated in four directions by the light sources 34N, 34S, 34E and 34W below the divided package TP. Note that it is preferable that the divided package TP be not irradiated with light other than the light emitted from the light sources 34 during imaging.

As shown in Figure 3, the distances (d1, d2, d3 and d4) between the respective light sources 34N, 34S, 34E and 34W above the divided package TP and the imaging optical axis PA of the camera 36A are the same as each other. That is, the light sources 34 are apart from the imaging optical axis PA by the same distance (d1 = d2 = d3 = d4). The light sources 34N, 34S, 34E and 34W below the divided package TP and the camera 36B are also arranged in a similar manner.

The medicine inspection support device 1 may be provided with a stage on which the divided packages TP are placed. In this case, the stage may be made of a material that transmits the light emitted from the light sources 34N, 34S, 34E and 34W below the divided package TP.

The prescription reader 38 shown in Figure 1 (example of prescription information obtaining unit) reads a prescription and obtains prescription information. For example, information on a patient's name, prescribed medicines and their quantities and the like are read from a prescription written on a paper through OCR (Optical Character Recognition). If a bar code or the like indicating information pertaining to the prescribed medicines is recorded on the prescription, information on the prescribed medicines and their quantities and the like may be read from the bar code. Alternatively, a user may read a prescription, and input prescription information through an input device, such as a keyboard, included in the operation unit 60. The prescription information obtained by the prescription reader 3 8 is stored in the storage unit 40.

Here, reading the prescription to obtain the prescription information has been described as an example. Alternatively, the prescription reader 38 can obtain various kinds of prescription data instead of the prescription information. The prescription data may be information itself described on the prescription, or information set and/or changed by a doctor and/or a pharmacist based on the information described on the prescription. For example, the prescription data can also include information that a generic medicine has been selected in a case where an original medicine is described on the prescription, information that an original medicine or a generic medicine has been selected in a case where only a common name of a medicine is described on the prescription, and so on.

The storage unit 40 is a medicine database that includes a non-transitory recording medium, such as a CD (Compact Disk), a DVD (Digital Versatile Disk), a hard disk, and various semiconductor memories. In addition to the prescription information and the master images, the taken images obtained by the cameras 36A and 36B or processed taken images (images based on taken images) which have been subjected to image processing are stored in the storage unit 40 in association with each other.

The display unit 50 includes a monitor 52, and can display the prescription information obtained from the prescription reader 38, the taken images of the medicines packaged in divided packages, the master images stored in the storage unit 40 and the like. The operation unit 60 includes a pointing device, such as a mouse, and an input device such as a keyboard. The user can operate images, buttons and the like displayed on the monitor 52 through the operation unit 60.

The conveyance mechanism 70 conveys the divided packages TP (strip medicine package PB) in the +x direction in Figure 3.

### [Configuration of processing unit]

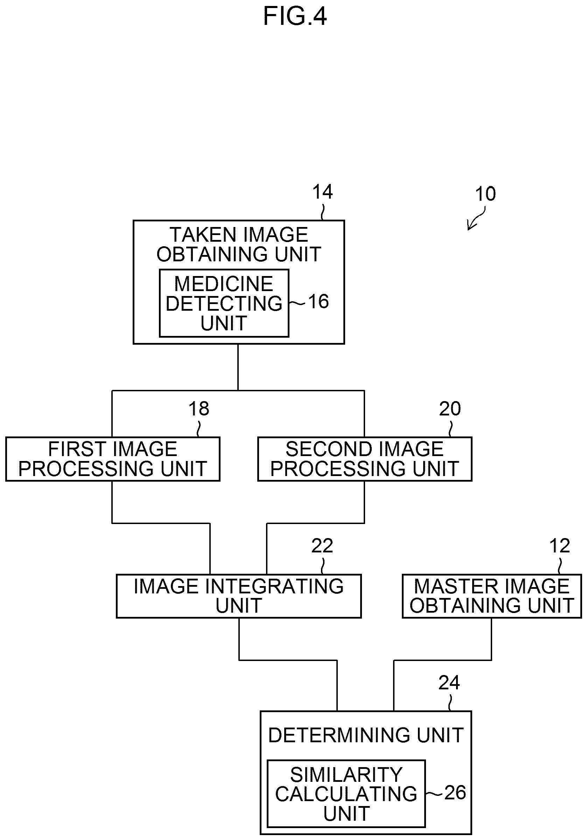

Figure 4 shows the internal configuration of the processing unit 10 (example of image processing device). As shown in Figure 4, the processing unit 10 includes a master image obtaining unit 12, a taken image obtaining unit 14, a first image processing unit 18, a second image processing unit 20, an image integrating unit 22 and a determining unit 24.

The master image obtaining unit 12 reads and obtains the master image of the prescribed medicine from the storage unit 40 (example of master image storage unit) based on the prescription information obtained from the prescription reader 38. The master image is a reference image of a medicine, or a processed image obtained by applying image processing to the reference image. The master image is represented such that a luminance value of the identification information is relatively high in comparison with values of the other portions. The master images include a pair of master images that are a master image on one side (for example, front side) and a master image on the other side (for example, rear side) for one medicine, and are preliminarily stored in the storage unit 40 in association with each other.

The taken image obtaining unit 14 (example of imaging control unit) controls the illuminating unit 32 and the cameras 36A and 36B, and obtains a plurality of taken images where the light illumination directions to the surface of each medicine packaged in the divided package TP are different from each other. The taken image obtaining unit 14 further includes a medicine detecting unit 16. The medicine detecting unit 16 detects an area (areas) where the medicine is taken, from each of the obtained taken images.

The first image processing unit 18 performs a process of enhancing an engraved mark portion of a medicine based on at least one taken image among the taken images obtained by the taken image obtaining unit 14, and generates a first enhanced image. In a case where the first enhanced image is generated based on a plurality of taken images, the process of enhancing the engraved mark portion of the medicine is applied to each taken image, and then the processed taken images are composed by addition or multiplication.

The second image processing unit 20 performs a process of enhancing a printed character portion of a medicine based on at least one taken image among the taken images obtained by the taken image obtaining unit 14, and generates a second enhanced image. In a case where the second enhanced image is generated based on the taken images, the process of enhancing the printed character portion of the medicine is applied to each taken image, and then the processed images are composed by addition or multiplication.

The image integrating unit 22 integrates the first enhanced image generated by the first image processing unit 18 and the second enhanced image generated by the second image processing unit 20, and generates an integrated image as a collation target image (image to be collated).

The determining unit 24 collates the integrated images generated by the image integrating unit 22 with the master images obtained by the master image obtaining unit 12, and determines whether the medicine (medicines) to be dispensed in conformity with the prescription is the same as the medicine (medicines) packaged in the divided package TP or not.

The determining unit 24 includes a similarity calculating unit 26. The similarity calculating unit 26 calculates the similarity indicating a degree at which the integrated images generated by the image integrating unit 22 are similar to the master images obtained by the master image obtaining unit 12. The determining unit 24 collates the integrated images with the master images based on the similarity.

The detailed processes of the medicine inspection support method according to these functions are described later.

### [Processes of medicine inspection support method]

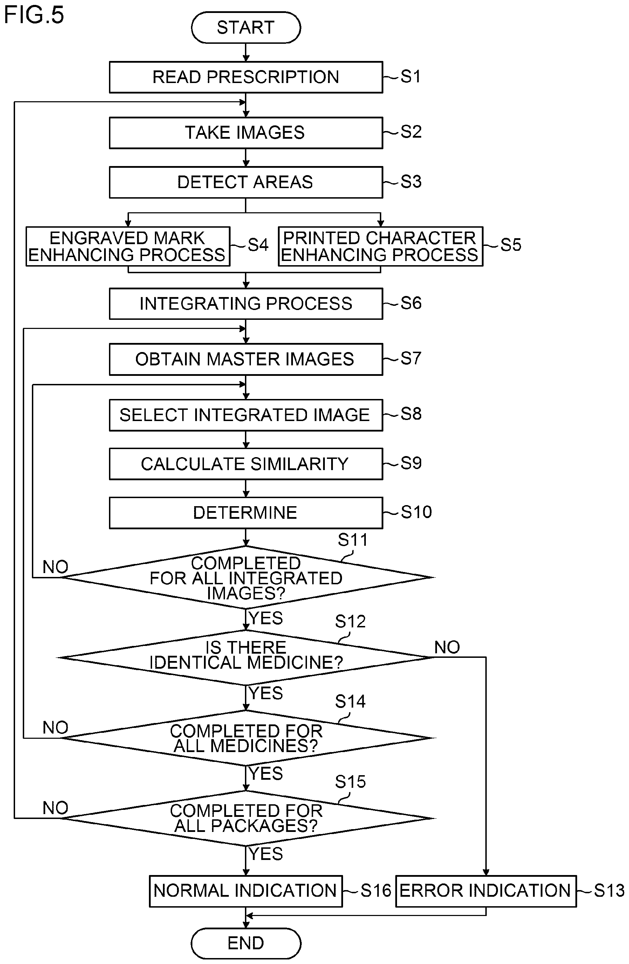

A medicine inspection support method (example of image processing method) by the medicine inspection support device 1 is described. Figure 5 is a flowchart showing the processes of the medicine inspection support method.

In step S1, a prescription is read by the prescription reader 38 to obtain the prescription information. Prescription information preliminarily stored in the storage unit 40 may be used.

In step S2 (example of taken image obtaining step, and example of taken image obtaining function), the taken image obtaining unit 14 controls the illuminating unit 32 and the camera 36A and the camera 36B to image medicines (example of second medicine) packaged in the divided package TP of the strip medicine package PB and obtain a plurality of taken images.

Here, the taken image obtaining unit 14 sequentially turns on the light sources 34N, 34S, 34E and 34W above the divided package TP one by one and turns off the others to thus sequentially switch the illumination direction in which the divided package TP is irradiated, and causes the camera 36A to image the divided package TP repeatedly every time the illumination direction is switched. Accordingly, the taken image obtaining unit 14 obtains taken images (taken images of second medicine) of the upper side of the divided package TP, which are taken with four illumination directions different from each other.

The taken image obtaining unit 14 sequentially turns on the light sources 34N, 34S, 34E and 34W below the divided package TP one by one and turns off the others to thus sequentially switch the illumination direction in which the divided package TP is irradiated, and causes the camera 36B to image the divided package TP repeatedly every time the illumination direction is switched. Accordingly, the taken image obtaining unit 14 obtains taken images of the lower side of the divided package TP, which are taken with four illumination directions different from each other.

Furthermore, it may be possible to image the divided package TP by the camera 36A in a state where all the light sources 34N, 34S, 34E and 34W (example of two or more light sources) above the divided package TP are turned on, to obtain a taken image (full-lighting image). Alternatively, it may be possible to image the divided package TP by the camera 36B in a state where all the light sources 34N, 34S, 34E and 34W below the divided package TP are turned on, to obtain a taken image.

In step S3, the medicine detecting unit 16 detects areas (medicine areas) where medicines are imaged, from each of the taken images obtained by the taken image obtaining unit 14. Because a plurality of medicines are packaged in the divided package TP, a plurality of medicine areas are detected from each taken image. By detecting the medicine areas, the areas to be processed can be limited, which can reduce the time required to the processes. Here, regarding the medicine areas of the same medicine, a medicine area detected from the taken image through the camera 36A and a medicine area detected from the taken image through the camera 36B are associated with each other as a pair of medicine areas of the same medicine.

In step S4 (example of first image processing step, and example of first image processing function), the first image processing unit 18 performs the process of enhancing engraved mark portions of the medicines, based on at least one taken image among the taken images obtained by the taken image obtaining unit 14. Here, for each pair of medicine areas of the same medicine among the medicine areas detected in step S3, a corresponding pair of first enhanced images is generated. A plurality of pairs of first enhanced images thus generated, are input into the image integrating unit 22.

As the process of enhancing the engraved mark portion of the medicine, for example, three-dimensional information on the surface of the medicine is obtained by photometric stereo or the like, and the first enhanced images are generated. For example, contour extraction images that represent heights in grayscale (luminance) in the images, which are described in PTL 1, can be adopted as the first enhanced images. Here, the first enhanced images are represented to have high luminance values of pixels at portions of grooves of the engraved marks of the medicines.

In step S5 (example of second image processing step, and example of second image processing function), the second image processing unit 20 performs the process of enhancing the printed character portions of the medicines, based on at least one taken image among the taken images obtained by the taken image obtaining unit 14. Here, for each pair of medicine areas of the same medicine among the medicine areas detected in step S3, a corresponding pair of second enhanced images is generated. A plurality of pairs of second enhanced images thus generated, are input into the image integrating unit 22.

As the process of enhancing the printed character portion of the medicine, for example, at least one of a smoothing process for reducing the noise component, a sharpening process for enhancing the edge, and an edge detecting process for removing the portions other than the printed character portion is applied to perform a binarizing process. Here, it possible to use any of publicly known methods such as a process of adopting, as a pixel value, an absolute value of a difference from the average value of pixel values, a process of adopting, as a pixel value, a result of division by the average value of pixel values, a process of division by a blurred image, and a process of applying the difference of local pixel values. Here, the second enhanced images are represented to have high luminance values of pixels at printed character portions of the medicines.

In step S6 (example of image integrating step, and example of image integrating function), the image integrating unit 22 compares luminance values of pixels of the first enhanced image and the second enhanced image generated from the same taken image at corresponding positions with each other, adopts the luminance values of pixels being relatively higher to generate an integrated image. Here, a pair of integrated images is generated from a pair of first enhanced images and a pair of second enhanced images generated from the same medicine area. A plurality of pairs of integrated images thus generated, are input into the determining unit 24.

In step S7 (example of master image obtaining step, and example of master image obtaining function), a pair of master images for one medicine among the medicines (example of first medicine) included in the prescription information obtained in step S1, is obtained. It may be possible to obtain reference images stored in the storage unit 40 as they are to adopt them as the master images. Alternatively, it may be possible to apply image processing such as enlargement or reduction, or brightness adjustment, to the reference images and adopted them as the master images. As described above, a pair of master images exists for one medicine.

In step S8, the determining unit 24 selects a pair of integrated images among the pairs of integrated images generated in step S6.

In step S9, the similarity calculating unit 26 calculates the similarity between the pair of master images obtained in step S7 and the pair of integrated images selected in step S8. The similarity described here is calculated such that the greater the degree of the similarity is, the higher the value of the similarity is.

In step S10 (example of determining step, and example of determining function), the determining unit 24 determines whether or not the imaged medicine (second medicine) is the same as one of the medicines (first medicine) described in the prescription based on the similarity calculated in step S9. Here, if the similarity is higher than a predetermined threshold, they are determined to be the same.

Alternatively, it may be possible to calculate the similarities with all the integrated images, for the pair of master images obtained in step S7, and then determine that, for the integrated image having the highest similarity, the medicine in that integrated images is the same as the medicine in the master images.

In step S11, the processing unit 10 determines whether or not collation is completed for all the integrated images. If there are any integrated images having not been collated yet, the processing returns to step S8, new integrated images are selected and analogous processes are repeated. If the collation is completed, the processing transitions to step S12.

In step S12, it is determined whether or not there is a medicine which is the same as (identical to) the medicine in the master images selected in step S7, that is, whether or not there is a medicine which is the same as one of medicines described in the prescription. If it is determined that there is no medicine which is the same as one of the medicines described in the prescription, the processing transitions to step S13. If it is determined that there is a medicine which is the same as one of the medicines described in the prescription, the processing transitions to step S14.

In step S13, the display unit 50 displays that there is no medicine which is the same as one of the medicines described in the prescription, on the monitor 52 (error indication), and the processing of this flowchart is finished.

In step S14, the processing unit 10 determines whether or not collation is completed for all the medicines described in the prescription (all the medicines included in the prescription information read in step S1). If there is any medicine having not been collated yet, the processing returns to step S7, a new pair of master images is obtained and analogous processes are repeated. If the collation is completed, the processing transitions to step S15.

In step S15, the processing unit 10 determines whether or not collation is completed for all the divided packages TP included in the strip medicine package PB. If there is a divided package TP which has not been collated yet, the processing returns to step S2, the strip medicine package PB is conveyed, taken images of a new divided package TP are obtained, and analogous processes are repeated. If the collation is completed for all the divided packages TP, the processing transitions to step S16.

In step S16, the display unit 50 displays that the audit result is normal (normal indication) on the monitor 52, and the processing of this flowchart is finished.

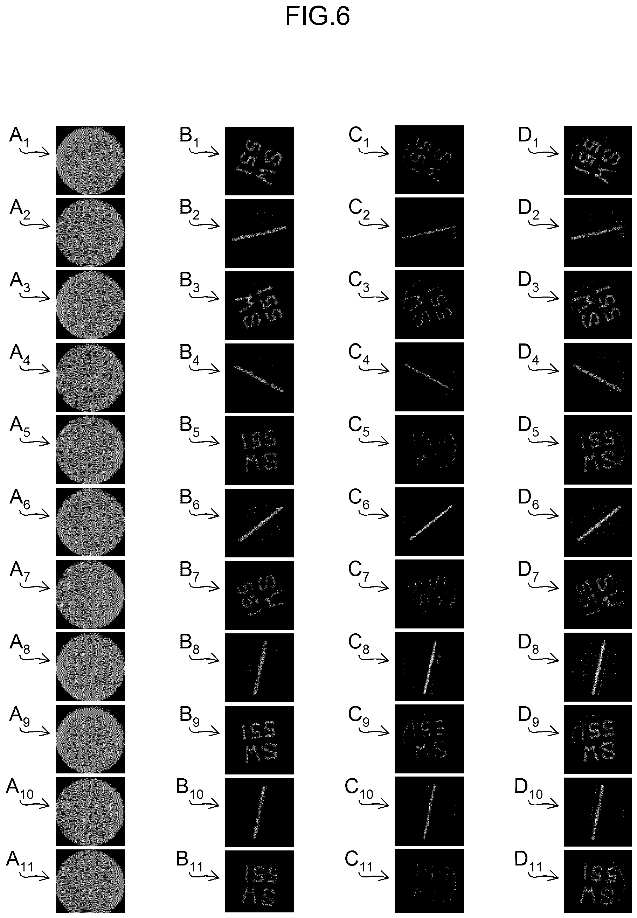

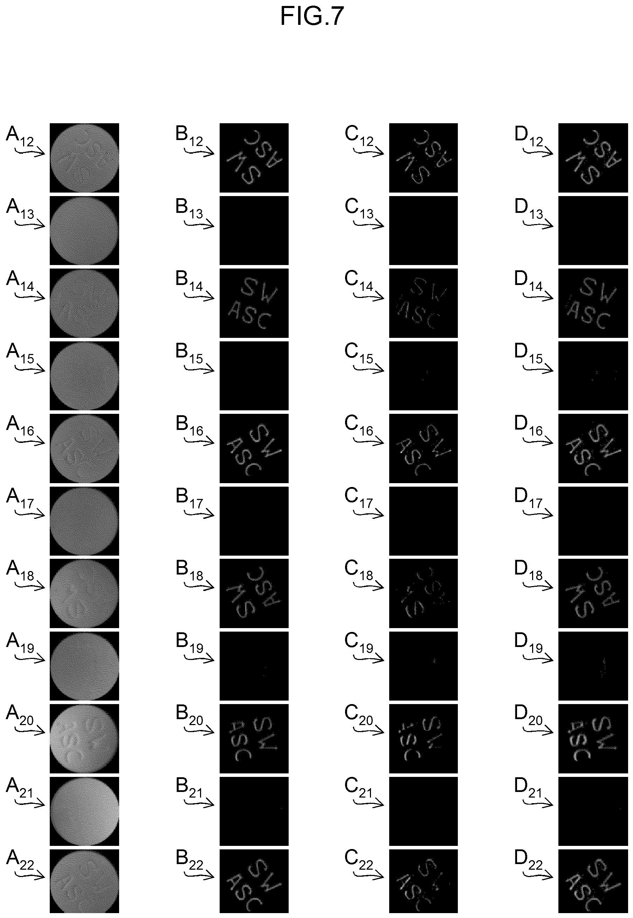

Figures 6 to 9 show examples of image processing results. Medicine areas A₁ to An shown in Figure 6 are examples of medicine areas detected from taken images of a medicine having an engraved mark on its surface. Likewise, medicine areas A₁₂ to A₂₂ shown in Figure 7 are examples of medicine areas detected from taken images of a medicine having an engraved mark on its surface.

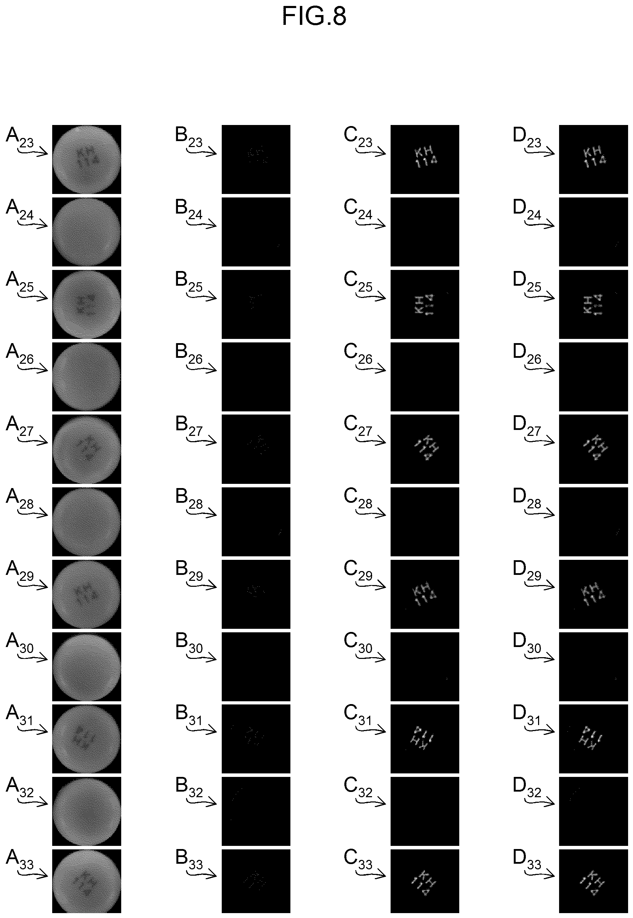

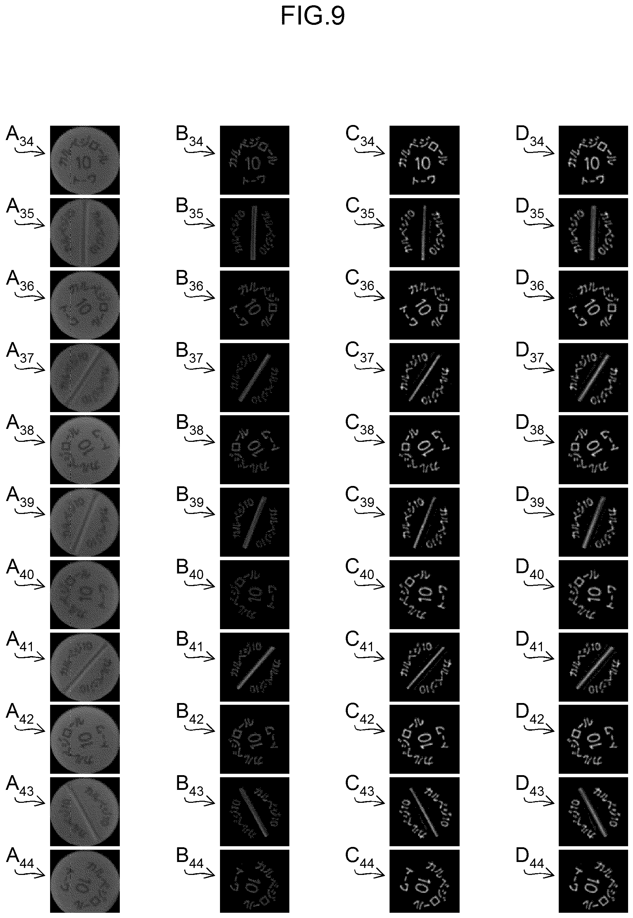

Medicine areas A₂₃ to A₃₃ shown in Figure 8 are examples of medicine areas detected from taken images of a medicine having printed characters on its surface. Likewise, medicine areas A₃₄ to A₄₄ shown in Figure 9 are examples of medicine areas detected from taken images of a medicine having printed characters on its surface.

Note that the taken images where the medicine areas A₁ to A₄₄ shown in Figures 6 to 9 are full-lighting images which are obtained in a state where all the light sources 34N, 34S, 34E and 34W are turned on. The medicine areas A₁ to A₄₄ include medicine areas obtained from taken images on one surface side of the medicine, and medicine areas obtained from taken images on the other surface side, in a mixed manner.

First enhanced images B₁ to B₄₄ shown in Figures 6 to 9 are examples of the first enhanced images respectively generated from the same areas as the medicine areas A₁₁ to A₄₄. Here are shown the first enhanced images generated based on medicine areas in four (example of three or more) taken images taken in a state where the light sources 34N, 34S, 34E and 34W are individually turned on.

Second enhanced images C₁ to C₄₄ shown in Figures 6 to 9 are examples of the second enhanced images generated from the respective medicine areas A₁₁ to A₄₄.

Integrated images D₁ to D₄₄ shown in Figures 6 to 9 are examples of integrated images generated from the first enhanced images B₁ to B₄₄ and the second enhanced images C₁ to C₄₄. For the medicines shown in Figures 6 and 7, the luminance values of the identification information are represented to be relatively larger (whiter) in the first enhanced images B₁ to B₂₂ than in the second enhanced images C₁ to C₂₂. Accordingly, the pixels of the first enhanced images B₁ to B₂₂ are mainly adopted as the integrated images.

On the other hand, for the medicines shown in Figures 8 and 9, the luminance values of the identification information are represented to be relatively larger in the second enhanced images C₂₃ to C₄₄ than in the first enhanced images B₂₃ to B₄₄. Accordingly, the pixels of the second enhanced images C₂₃ to C₄₄ are mainly adopted as the integrated images.

In this embodiment, the first image processing unit 18 performs the process of increasing the luminance values of pixels of the engraved mark portion of the medicine, the second image processing unit 20 performs the process of increasing the luminance values of pixels of the printed character portion of the medicine, and the image integrating unit 22 compares the luminance values of pixels of the first enhanced image and the second enhanced image at corresponding positions, and adopts the luminance value of the pixels of a higher luminance. In the case where the luminance value of the identification information in the master image is represented to be relatively lower than the values of the other portions, the first image processing unit 18 may perform the process of reducing the luminance value of the pixels of the engraved mark portion of the medicine, the second image processing unit 20 may perform the process of reducing the luminance value of the pixels of the printed character portion of the medicine, and the image integrating unit 22 may compare the luminance values of the pixels of the first enhanced image and the second enhanced image at corresponding positions, and adopt the luminance value of the pixels of a lower luminance.

In this embodiment, the first enhanced image and the second enhanced image are generated for the areas detected by the medicine detecting unit 16. Alternatively, it may be possible to generate the first enhanced images and the second enhanced images for the taken images, and then detect the medicine areas.

As described above, the process of enhancing the engraved mark portion of the dispensed second medicine is performed to generate the first enhanced images, and the process of enhancing the printed character portion of the second medicine is performed to generate the second enhanced images, the first enhanced images and the second enhanced images are integrated to generate the integrated images, and the integrated images are used to collate the first medicine to be dispensed with the master images. Accordingly, irrespective of whether the identification information added to the second medicine is the engraved mark or the printed character, the identification information can be appropriately recognized, and correct collation can be achieved.

### <Second embodiment>

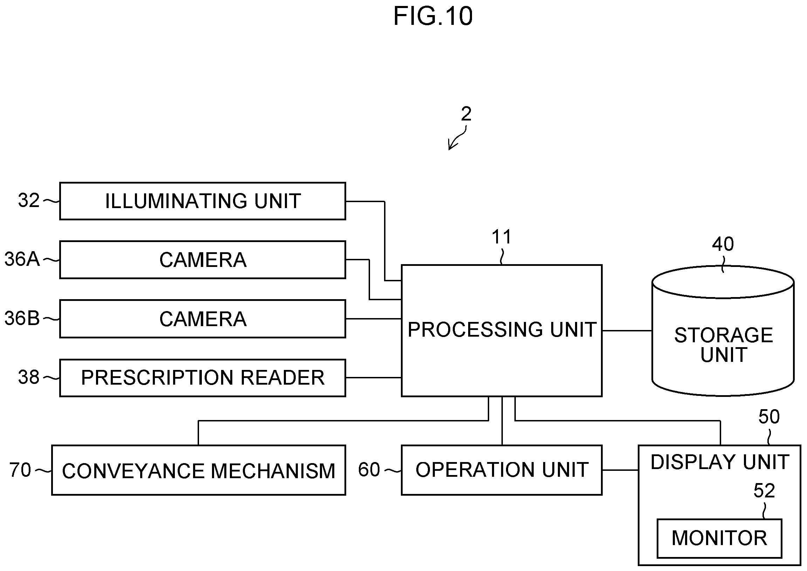

Figure 10 shows the configuration of a medicine inspection support device 2 according to a second embodiment. Note that the parts common to those of the medicine inspection support device 1 shown in Figure 1 are assigned the same numerals or characters; their detailed description is omitted. The medicine inspection support device 2 includes a processing unit 11. An illuminating unit 32, a camera 36A, a camera 36B, and a prescription reader 38 are connected to the processing unit 11.

The illuminating unit 32 includes multiple light sources 34. The arrangement of the light sources 34, the camera 36A and the camera 36B is analogous to that of the medicine inspection support device 1 shown in Figures 2 and 3.

### [Configuration of processing unit]

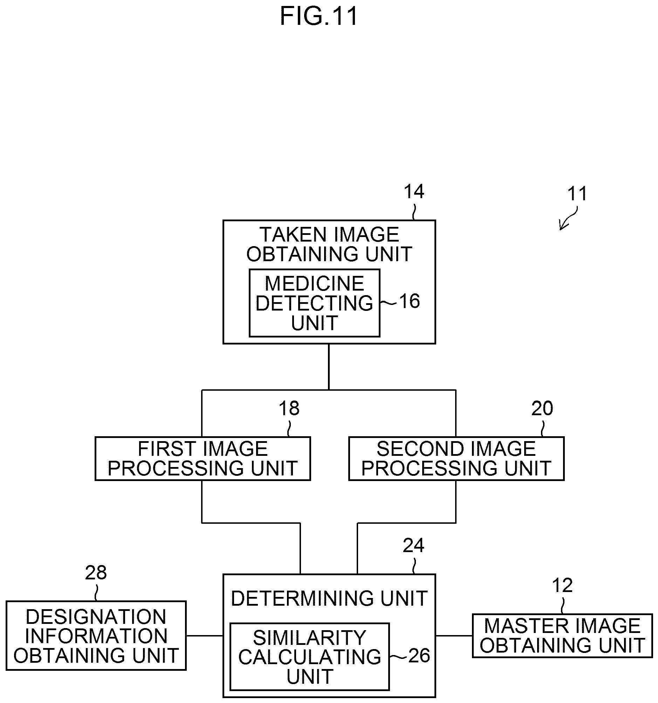

Figure 11 shows the internal configuration of the processing unit 11 (example of image processing device). Note that the parts common to those of the processing unit 10 shown in Figure 4 are assigned the same numerals or characters; their detailed description is omitted. As shown in Figure 11, the processing unit 11 includes a designation information obtaining unit 28.

Designation information is information that designates, on a medicine-by-medicine basis, which one of the first enhanced image generated by the first image processing unit 18 and the second enhanced image generated by the second image processing unit 20 is to be used for collation.

In a case where identification information has been added to a medicine with mark engraving, discrimination of the identification information is facilitated by using the first enhanced images obtained by applying the process of enhancing the engraved mark portion of the medicine. Accordingly, the designation information associated with the master image of the medicine with the engraved mark designates the first enhanced images as the collation target image to be used for collation. In aa case where identification information has been added to a medicine with character printing, discrimination of the identification information is facilitated by using the second enhanced image obtained by applying the process of enhancing the printed character portion of the medicine. Accordingly, the designation information associated with the master image of the medicine with the printed character designates the second enhanced image as the collation target image to be used for collation.

The designation information is stored in the storage unit 40 in association with the information on the medicine, such as the master image. The designation information obtaining unit 28 reads and obtains the designation information on the prescribed medicine from the storage unit 40 based on the prescription information obtained from the prescription reader 38.

The first enhanced image generated by the first image processing unit 18 and the second enhanced image generated by the second image processing unit 20 are input into the determining unit 24. The determining unit 24 selects either the first enhanced images or the second enhanced images as the collation target images based on the designation information obtained by the designation information obtaining unit 28. The determining unit 24 collates the selected collation target images with the master images obtained by the master image obtaining unit 12, and determines whether or not the medicines packaged in the divided package TP are the same as the medicines to be dispensed in accordance with the prescription.

### [Processes of medicine inspection support method]

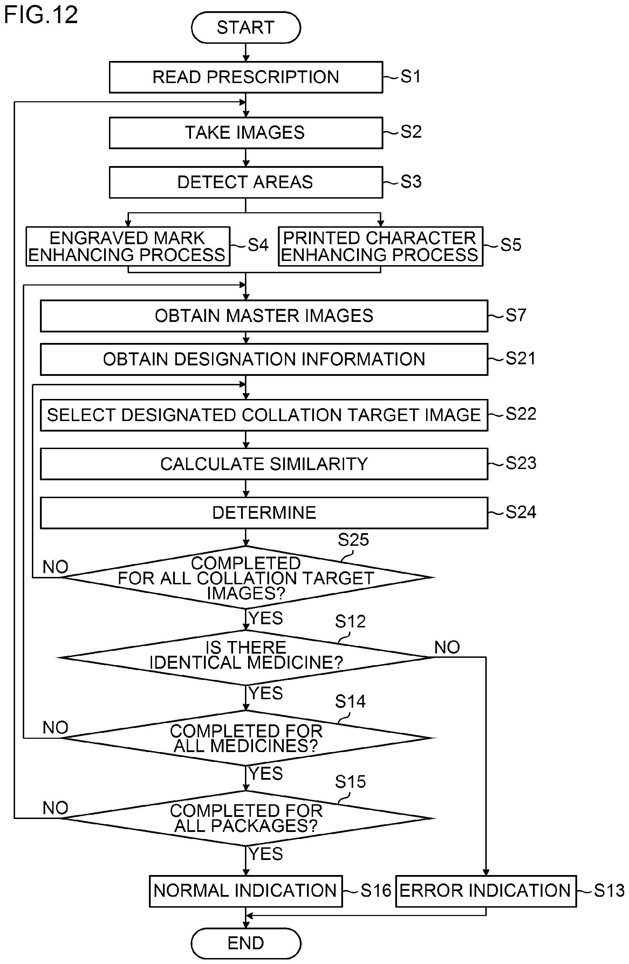

A medicine inspection support method (example of image processing method) by the medicine inspection support device 2 is described. Figure 12 is a flowchart showing the processes of the medicine inspection support method. Note that the parts common to those of the flowchart shown in Figure 5 are assigned the same numerals or characters; their detailed description is omitted.

In step S1, the prescription information is obtained.

In step S2 (example of taken image obtaining step, and example of taken image obtaining function), for each of the upper side and the lower side of the divided package TP, taken images are obtained. The obtained taken images include images respectively taken in four illumination directions different from each other and a full-lighting image taken while irradiating from all the four directions.

In step S3, the medicine areas are detected from each of the taken images. Here, a medicine area detected from the taken image through the camera 36A and a medicine area detected from the taken image through the camera 36B, which are medicine areas of the same one medicine, are detected as a pair of medicine areas of the same medicine and associated with each other.

In step S4 (example of first image processing step, and example of first image processing function), the process of enhancing the engraved mark portion of the medicine is performed to generate the first enhanced image of each medicine area. The first image processing unit 18 generates a pair of first enhanced images for the pair of the medicine areas of the same medicine. Here, the first enhanced images are represented to have high luminance values of pixels at a portion of a groove of the engraved mark of the medicine. The plurality of pairs of first enhanced images thus generated, are input into the determining unit 24.

In step S5 (example of second image processing step, and example of second image processing function), the process of enhancing the printed character portion of the medicine is performed to generate the second enhanced image of each medicine area. The second image processing unit 20 generates a pair of second enhanced images for the pair of the medicine areas of the same medicine. Here, the second enhanced images are represented to have high luminance values of pixels at a printed character portion of the medicine. The plurality of pairs of second enhanced images thus generated, are input into the determining unit 24.

In step S7 (example of master image obtaining step, and example of master image obtaining function), a pair of master images of one medicine among the medicines included in the prescription information obtained in step S 1 is obtained. The master images are represented to have relatively high luminance value of the identification information in comparison with the values of the other portions.

In step S21 (example of designation information obtaining step, and example of designation information obtaining function), the designation information obtaining unit 28 obtains the designation information associated with the master images obtained in step S7, from the storage unit 40. The designation information designates either the first enhanced images or the second enhanced images.

In step S22, the determining unit 24 selects the images designated by the designation information input from the designation information obtaining unit 28, between the first enhanced images and the second enhanced images of the medicine to be collated. That is, if the designation information designates the first enhanced images, the pair of first enhanced images is selected as the pair of collation target images (images to be collated); if the designation information designates the second enhanced images, the pair of second enhanced images is selected as the pair of collation target images. Note that in a case where an engraved mark is added on one surface of the medicine and a printed character is added on the other surface, the designation information may designate both the first enhanced images and the second enhanced images. In this case, it may be possible to appropriately select a required first enhanced image and second enhanced image from the pair of first enhanced images and the pair of second enhanced images.

In step S23, the similarity calculating unit 26 calculates the similarity indicating the degree at which the pair of master images obtained in step S7 and the pair of collation target images selected in step S22 are similar to each other. The similarity described here is calculated such that the greater the degree of the similarity is, the higher the value of the similarity is.

In step S24 (example of determining step, and example of determining function), the determining unit 24 determines whether or not the imaged medicine is the same as one of the medicines described in the prescription based on the similarity calculated in step S23. Here, if the similarity is higher than a predetermined threshold, they are determined to be the same.

In step S25, the processing unit 10 determines whether or not collation is completed for all the collation target images. If there are integrated images having not been collated yet, the processing returns to step S22, new collation target images are selected and analogous processes are repeated. If the collation is completed, the processing transitions to step S12.

In step S12, it is determined whether or not there is a medicine which is the same as (identical to) the medicine in the master image selected in step S7, that is, whether or not there is a medicine which is the same as one of the medicines described in the prescription. If it is determined that there is no medicine which is the same as one of the medicines described in the prescription, the processing transitions to step S13. If it is determined that there is a medicine which is the same as one of the medicines described in the prescription, the processing transitions to step S14.

In step S13, the display unit 50 displays that there is no medicine which is the same as one of the medicines described in the prescription, on the monitor 52 (error indication), and the processing of this flowchart is finished.

In step S14, the processing unit 10 determines whether or not collation is completed for all the medicines described in the prescription (all the medicines included in the prescription information read in step S1). If there is any medicine having not been collated yet, the processing returns to step S7, new master images are obtained and analogous processes are repeated. If the collation is completed, the processing transitions to step S15.

In step S15, the processing unit 10 determines whether or not collation is completed for all the divided packages TP included in the strip medicine package PB. If there is any divided package TP having not been collated yet, the processing returns to step S2, the strip medicine package PB is conveyed, taken images of a new divided package TP are obtained, and analogous processes are repeated. If the collation is completed, the processing transitions to step S16.

In step S16, the display unit 50 displays that the audit result is normal (normal indication) on the monitor 52, and the processing of this flowchart is finished.

The medicines shown in Figures 6 and 7 have engraved marks added thereto. The designation information associated with each master image of the medicine designates the first enhanced images. Accordingly, for collation of the master images relating to the medicine areas A₁ to A₂₂, the respective first enhanced images B₁ to B₂₂ are selected.

Meanwhile, the medicines shown in Figures 8 and 9 have printed characters added thereto. The designation information associated with each master image of the medicine designates the second enhanced images. Accordingly, for collation of the master images relating to the medicine areas A₂₃ to A₄₄, the respective second enhanced images C₂₃ to C₄₄ are selected.

In this embodiment, the first image processing unit 18 performs the process of increasing the luminance values of the pixels of the engraved mark portion of the medicine, and the second image processing unit 20 performs the process of increasing the luminance values of the pixels of the printed character portion of the medicine. In a case where the luminance value of the identification information in the master image is represented to be relatively low with respect to the values of the other portions, it may be possible to perform the process of reducing the luminance values of the pixels of the engraved mark portion of the medicine by the first image processing unit 18, and perform the process of reducing the luminance values of the pixels of the printed character portion of the medicine by the second image processing unit 20.

As described above, the process of enhancing the engraved mark portion of the medicine is performed to generate the first enhanced images, and the process of enhancing the printed character portion of the medicine is performed to generate the second enhanced images, the designation information designating either the first enhanced images or the second enhanced images as images to be used for collation is obtained, and the images designated by the designation information are collated with the master images. Accordingly, irrespective of whether the identification information added to the medicine is the engraved mark or the printed character, the identification information can be appropriately recognized, and correct collation can be achieved.

Here, the designation information designates either the first enhanced images or the second enhanced images as images to be used for collation. Alternatively, it may be possible to determine whether the identification information is added by mark engraving or by character printing by analyzing the taken image or analyzing the first enhanced images and the second enhanced images, and then designate the images to be used for collation according to the determination result.

For example, the first enhanced images are compared with the second enhanced images. In a case where the luminance value of each pixel in the first enhanced images is higher, it can be determined that the identification information has been added by mark engraving. In a case where the luminance value of each pixel in the second enhanced imaged is higher, it can be determined that the identification information has been added by character printing.

Alternatively, it may be possible to analyze a correlation between the images respectively taken with four illumination directions different from each other, and then determine that the identification information has been added by mark engraving in a case where the correlation between the images is low, and determine that the identification information has been added by character printing in a case where the correlation between the images is high.

### <Third embodiment>

### [Processes of medicine inspection support method]

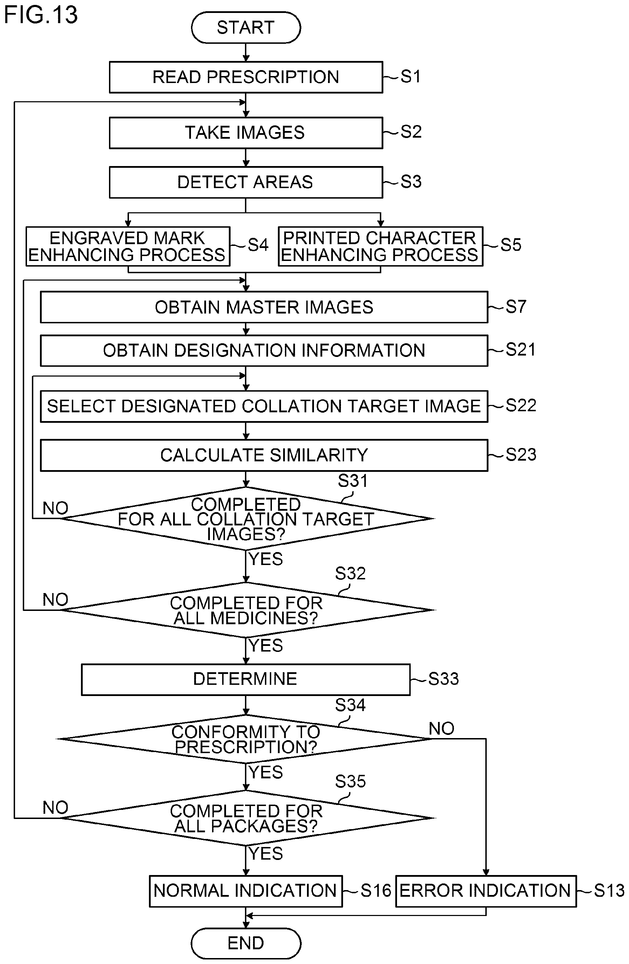

Figure 13 is a flowchart showing processes of a medicine inspection support method by the medicine inspection support device 2 according to the third embodiment. Note that the parts common to those of the flowchart shown in Figure 12 are assigned the same numerals or characters; their detailed description is omitted.

Steps S1 to S5 are analogous to those of the second embodiment. That is, the prescription information is obtained. The medicines packaged in a divided package TP of the strip medicine package PB are imaged to obtain a plurality of taken images. Medicine areas where the medicines are imaged are detected from the taken images. The first enhanced images where the engraved mark portion of the medicine are enhanced, and the second enhanced images where the printed character portion of the medicine are enhanced are generated.

In step S7, a pair of master images is obtained. In step S21, the designation information associated with the master images is obtained. Furthermore, in step S22, images designated by the designation information are selected from between the first enhanced images and the second enhanced images of the medicine to be collated.

In step S23, the similarity between the pair of master images obtained in step S7 and the pair of collation target images selected in step S22 is calculated. The similarity described here is calculated such that the greater the degree of the similarity is, the higher the value of the similarity is.

Next, in step S31, the processing unit 10 determines whether or not calculation of the similarity is completed for the combinations of all the collation target images with the master images obtained in step S7. If there is any collation target image whose similarity has not been calculated yet, the processing returns to step S22, a new pair of collation target images is selected and analogous processes are repeated. Accordingly, for the master images obtained in step S7, the similarities of the combinations with all the collation target images can be calculated. If the similarity calculation for the combinations with all the collation target images has been completed, the processing transitions to step S32.

Subsequently, in step S32, the processing unit 10 determines whether similarity calculation for all the medicines described in the prescription (all the medicines included in the prescription information read in step S1) is completed or not. If there is any medicine whose similarity calculation has not been completed yet, the processing returns to step S7, a new pair of master images is obtained and analogous processes are repeated. Accordingly, for every master image, the similarities of the combinations with all the collation target images obtained in step S22 can be calculated. If the similarity calculation for all the master images has been completed, the processing transitions to step S33.

In step S33 (example of determining step, and example of determining function), the determining unit 24 determines whether or not the imaged medicines are the same as the medicines described in the prescription based on the similarities of all the combinations between the master images and the collation target images calculated in step S23.

For example, for the four collation target images of the collation target image (1), the collation target image (2), the collation target image (3) and the collation target image (4), and the four master images of the master image (A), the master image (B), the master image (C) and the master image (D), it is assumed that the similarity (1A), the similarity (1B), the similarity (1C), the similarity (1D), the similarity (2A), the similarity (2B), ..., the similarity (4C) and the similarity (4D) have been calculated for each combinations.

Here, provided that the highest similarity among the similarities (1A, 1B, 1C and 1D) pertaining to the collation target image (1) is the similarity (1B), and the highest similarity among the similarities (1B, 2B, 3B and 4B) pertaining to the master image (B) is the similarity (1B), the determining unit 24 determines that the medicine indicated by the collation target image (1) is the medicine indicated by the master image (B).

Subsequently, the determining unit 24 excludes the collation target image (1) and the master image (B) from the collation targets, selects combinations having high similarities in an analogous manner, and determines combinations between the medicines described in the prescription and the imaged medicines.

In step S34, it is determined whether or not the medicines packaged in the divided package TP are as described in the prescription, from the determination result in step S33. If it is determined that the medicines do not conform to the prescription, the processing transitions to step S13. If it is determined that the medicines conform to the prescription, the processing transitions to step S35.

In step S13, the display unit 50 displays the error indication on the monitor 52, and the processing of this flowchart is finished.

In step S35, the processing unit 10 determines whether or not collation is completed of all the divided packages TP included in the strip medicine package PB. If there is any divided package TP having not been collated yet, the processing returns to step S2, the strip medicine package PB is conveyed, taken image of a new divided package TP are obtained, and analogous processes are repeated. If the collation is completed, the processing transitions to step S16.

In step S16, the display unit 50 displays the normal indication on the monitor 52, and the processing of this flowchart is finished.

As described above, the relative relationship of the similarities is used to determine the combination of the medicines to be dispensed and the dispensed medicines. Accordingly, it can be determined whether or not the medicines to be dispensed are the same as the dispensed medicines, for all the imaged medicines.

Likewise, it may be possible to determine whether or not the imaged medicines are the same as the medicines described the prescription in the first embodiment, based on the relative relationship of the similarities of all the combinations of the master images and the integrated images.

### <Fourth embodiment>

The support for auditing medicines has thus been described above. The present invention is applicable also to support for differentiating (identifying) medicines.

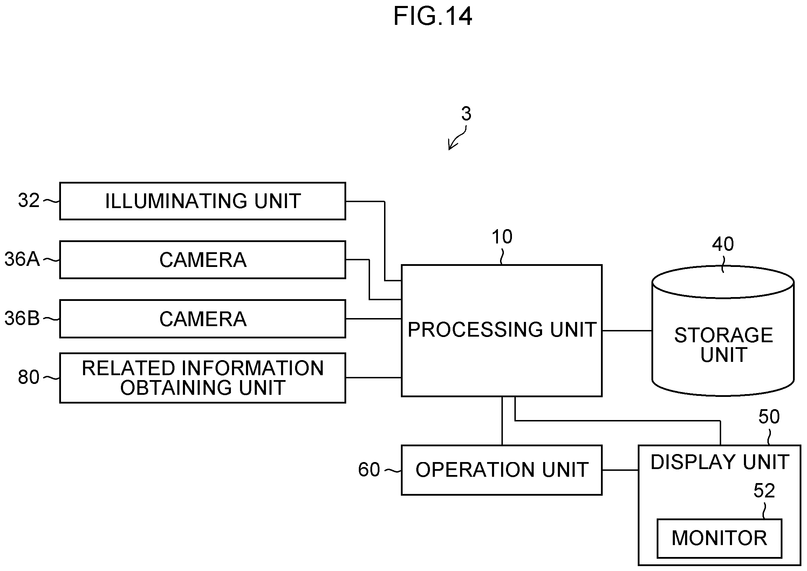

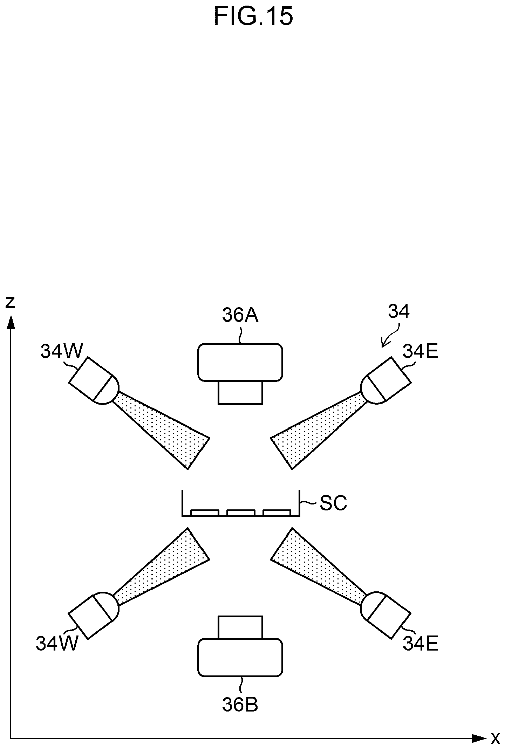

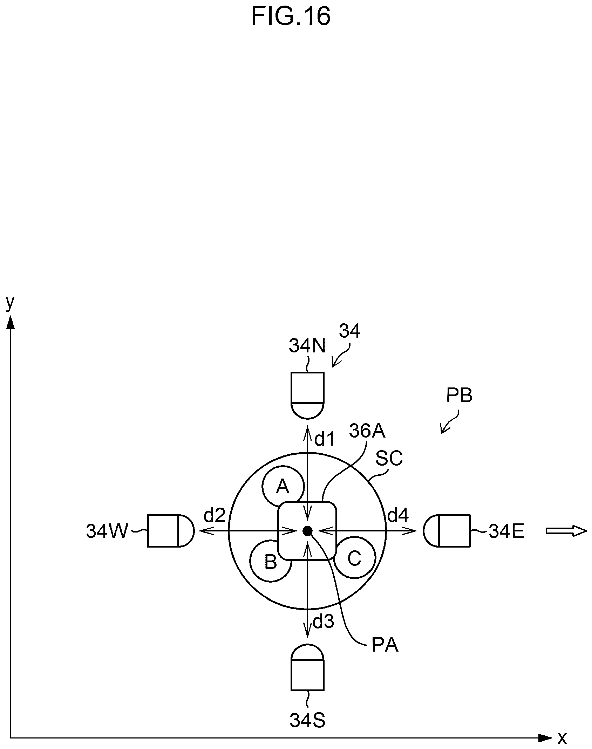

Figure 14 shows the configuration of a medicine inspection support device 3 according to a fourth embodiment. Note that the parts common to those of the medicine inspection support device 1 shown in Figure 1 are assigned the same numerals or characters; their detailed description is omitted. Figure 15 is a side view showing situations where the medicine inspection support device 3 obtains images of medicines. Figure 16 is a top view thereof.

In the medicine inspection support device 3, medicines are placed in a Petri dish SC, and the Petri dish SC is placed on a stage, not shown. Note that the medicines may be directly placed on the stage. Alternatively, the medicines may be placed on the Petri dish SC or the stage in a state where the medicines are packaged in a divided package. The Petri dish SC and the stage are made of a material that transmits the light emitted from the light sources 34N, 34S, 34E and 34W below the Petri dish SC.

Similar to the medicine inspection support device 1, the illuminating unit 32 includes a plurality of light sources 34. The light sources 34 irradiate with light, the upper side and the rear side of the medicines placed on the Petri dish SC. The camera 36A and the camera 36B image the upper side and the rear side of the medicines placed on the Petri dish SC.

A related information obtaining unit 80 shown in Figure 14 obtains information related to the medicines placed on the Petri dish SC. For example, characteristics of medicines (for example, medicine kinds about whether the medicines are tablets or capsules, shapes, colors, etc.) which are recognized by visual and the like, or information on medicine names, quantities, a dosing instruction and the like described in a notebook, or what is called "medicine notebook (medicine history handbook)," are obtained as related information according to an operation by the user. The related information may be prescription data. The related information obtained by the related information obtaining unit 80 is stored in the storage unit 40.

The other points of the configuration of the medicine inspection support device 3 are analogous to those of the medicine inspection support device 1.

### [Processes of medicine inspection support method]

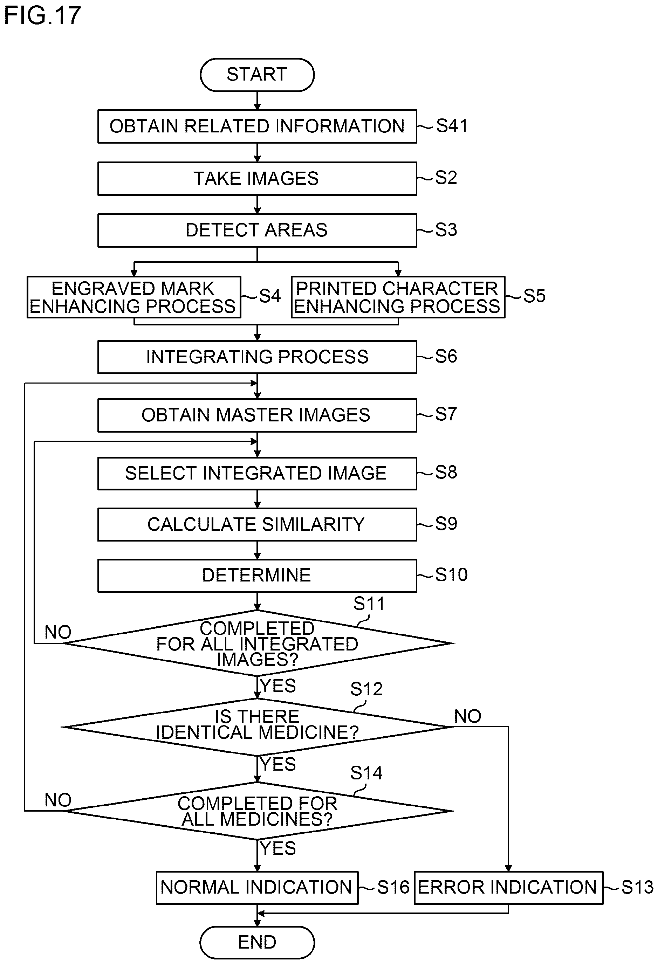

A medicine inspection support method by the medicine inspection support device 3 is described. Figure 17 is a flowchart showing the processes of the medicine inspection support method. Note that the parts common to those of the flowchart shown in Figure 5 are assigned the same numerals or characters; their detailed description is omitted.

In step S41, the related information obtaining unit 80 obtains the related information on all the medicines placed on the Petri dish SC through an operation by the user.

In subsequent step S2, the taken image obtaining unit 14 controls the illuminating unit 32, the camera 36A and the camera 36B to image the medicines placed on the Petri dish SC, thus obtaining a plurality of taken images. Here, for each of the upper side and the lower side of the Petri dish SC, images which are taken with four illumination directions different from each other, and a full-lighting image irradiated with light in all the four directions are obtained.

In step S3, the medicine detecting unit 16 detects areas where medicines are imaged, from each of the taken images obtained by the taken image obtaining unit 14. Here, regarding the medicine areas of the same medicine, a medicine area detected from the taken image through the camera 36A and a medicine area detected from the taken image through the camera 36B, are detected in association with each other as a pair of medicine areas of the same medicine.

In step S4, the first image processing unit 18 performs the process of enhancing the engraved mark portions of the medicines, based on at least one taken image among the taken images obtained by the taken image obtaining unit 14. In step S5, the second image processing unit 20 performs the process of enhancing the printed character portions of the medicines, based on at least one taken image among the taken images obtained by the taken image obtaining unit 14.

In step S6, the image integrating unit 22 compares the luminance values of the pixels of the first enhanced image and the second enhanced image generated from the same taken image at corresponding positions with each other, adopts the luminance values of the pixels being relatively higher and generates a pair of integrated images.

In step S7, the master image obtaining unit 12 of the processing unit 10 reads and obtains the master images of the medicine from the storage unit 40, based on the related information obtained by the related information obtaining unit 80.

In step S8, the determining unit 24 selects a pair of integrated images among the pairs of integrated images generated in step S6.

In step S9, the similarity calculating unit 26 calculates the similarity between the pair of master images obtained in step S7 and the pair of integrated images selected in step S8. The similarity described here is calculated such that the greater the degree of the similarity is, the higher the value of the similarity is.

In step S10, the determining unit 24 determines whether or not the imaged medicine is the same as the medicines whose related information have been obtained based on the similarity calculated in step S9.

In step S11, the processing unit 10 determines whether or not collation is completed for all the integrated images. If there are any integrated images having not been collated yet, the processing returns to step S8, new integrated images are selected and analogous processes are repeated. If the collation is completed, the processing transitions to step S12.

In step S12, it is determined whether or not there is a medicine which is the same as a medicine in the master images selected in step S7, that is, whether or not there is a medicine which is the same as one of the medicines whose related information have been obtained. If it is determined that there is no medicine which is the same as one of the medicines whose related information have been obtained, the processing transitions to step S13. If it is determined that there is a medicine which is the same as one of the medicines whose related information have been obtained, the processing transitions to step S16.

In step S13, the display unit 50 displays that there is no medicine which is the same as one of the medicines whose related information have been obtained, on the monitor 52 (error indication), and the processing of this flowchart is finished.

In step S14, the processing unit 10 determines whether collation is completed for all the medicines whose related information have been obtained or not. If there is any medicine having not been collated yet, the processing returns to step S7, a new pair of master images is obtained and analogous processes are repeated. If the collation is completed, the processing transitions to step S16.

In step S16, the display unit 50 displays that the differentiation result is normal (normal indication) on the monitor 52, and the processing of this flowchart is finished.

As described above, even in a case where the present invention is applied to a device which obtains related information on medicines and differentiates the medicines, the identification information can be appropriately recognized, and correct collation can be performed.

### <Fifth embodiment>

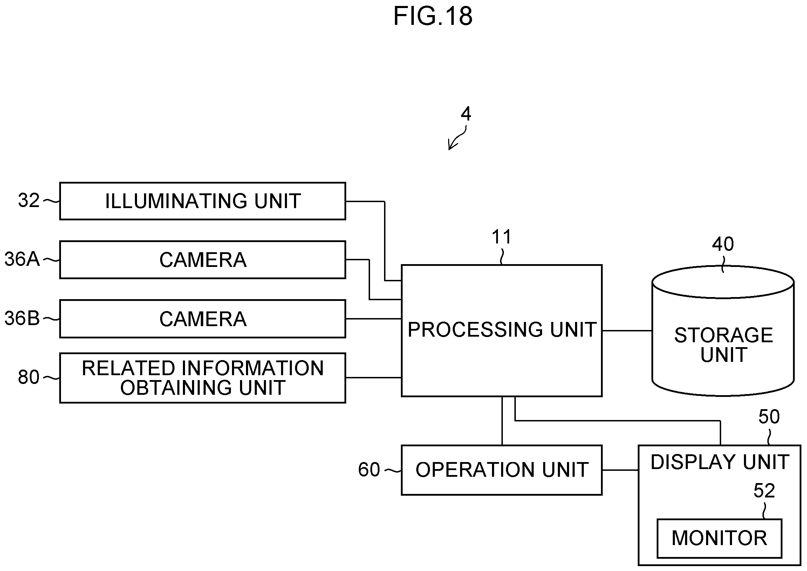

Figure 18 shows the configuration of a medicine inspection support device 4 according to a fifth embodiment. Note that the parts common to those of the medicine inspection support device 3 shown in Figure 14 are assigned the same numerals or characters; their detailed description is omitted.

The medicine inspection support device 4 includes a processing unit 11 instead of the processing unit 10 of the medicine inspection support device 3. The internal configuration of the processing unit 11 is analogous to that of the configuration shown in Figure 11.

### [Processes of medicine inspection support method]

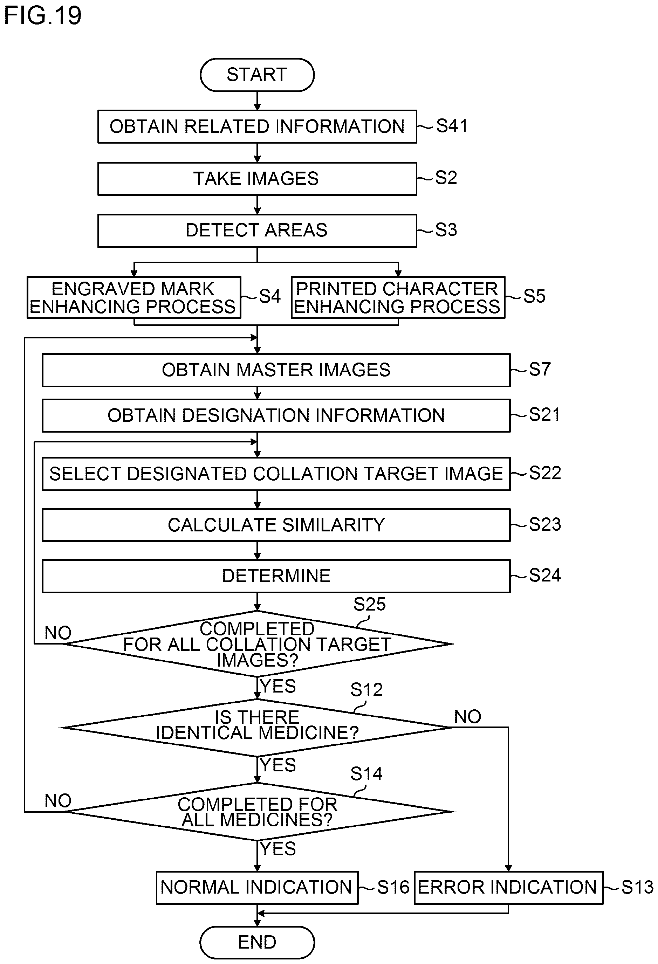

A medicine inspection support method by the medicine inspection support device 4 is described. Figure 19 is a flowchart showing the processes of the medicine inspection support method. Note that the parts common to those of the flowchart shown in Figure 12 are assigned the same numerals or characters; their detailed description is omitted.

In step S41, the related information obtaining unit 80 obtains the related information through an operation by the user.

In step S2, for each of the upper side and the lower side of the Petri dish SC, images which are taken with four illumination directions different from each other, and a full-lighting image irradiated with light in all the four directions are obtained.

In step S3, the medicine areas are detected from each of the taken images.

In step S4, the process of enhancing the engraved mark portion of the medicine is performed to generate a first enhanced image for each medicine area. In step S5, the process of enhancing the printed character portion of the medicine is performed to generate a second enhanced image for each medicine area.

In step S7, a pair of master images of one medicine among the medicines included in the related information obtained in step S1 is obtained. The master images are represented such that the luminance values of the identification information are relatively high in comparison with the values of the other portions.

In step S21, the designation information obtaining unit 28 obtains the designation information associated with the master images obtained in step S7, from the storage unit 40. The designation information designates either the first enhanced images or the second enhanced images.

In step S22, the determining unit 24 selects images designated by the designation information input from the designation information obtaining unit 28, from between the first enhanced images and the second enhanced images of the medicine to be collated. That is, if the designation information designates the first enhanced images, the pair of first enhanced images is selected as the pair of collation target images; if the information designates the second enhanced images, the pair of second enhanced images is selected as the pair of collation target images.

In step S23, the similarity calculating unit 26 calculates the similarity indicating the degree at which the pair of master images obtained in step S7 and the pair of collation target images selected in step S22 are similar to each other.

In step S24, the determining unit 24 determines whether or not the imaged medicine is the same as one of the medicines whose related information have been obtained based on the similarity calculated in step S23.

In step S25, the processing unit 10 determines whether collation is completed for all the collation target images or not. If there is any integrated image having not been collated yet, the processing returns to step S22, new collation target images are selected and analogous processes are repeated. If the collation is completed, the processing transitions to step S12.

In step S12, it is determined whether or not there is a medicine which is identical to a medicine in the master images selected in step S7, that is, whether or not there is a medicine which is identical to one of medicines whose related information have been obtained. If it is determined that there is no medicine which is the same as one of the medicines whose related information have been obtained, the processing transitions to step S13. If it is determined that there is any medicine which is the same as one of the medicines whose related information have been obtained, the processing transitions to step S14.

In step S13, the display unit 50 displays that there is no medicine which is the same as one of the medicines whose related information have been obtained, on the monitor 52 (error indication), and the processing of this flowchart is finished.

In step S14, the processing unit 10 determines whether or not collation is completed for all the medicines whose related information have been obtained. If there is any medicine having not been collated yet, the processing returns to step S7, new master images are obtained and analogous processes are repeated. If the collation is completed, the processing transitions to step S16.

In step S16, the display unit 50 displays that the differentiation result is normal (normal indication) on the monitor 52, and the processing of this flowchart is finished.

As described above, even in a case where the present invention is applied to a device which obtains related information on medicines and differentiates the medicines, the designation information is obtained and the images designated by the designation information are collated with the master images, thereby allowing the collation to be correctly performed.

### <Sixth embodiment>

### [Processes of medicine inspection support method]

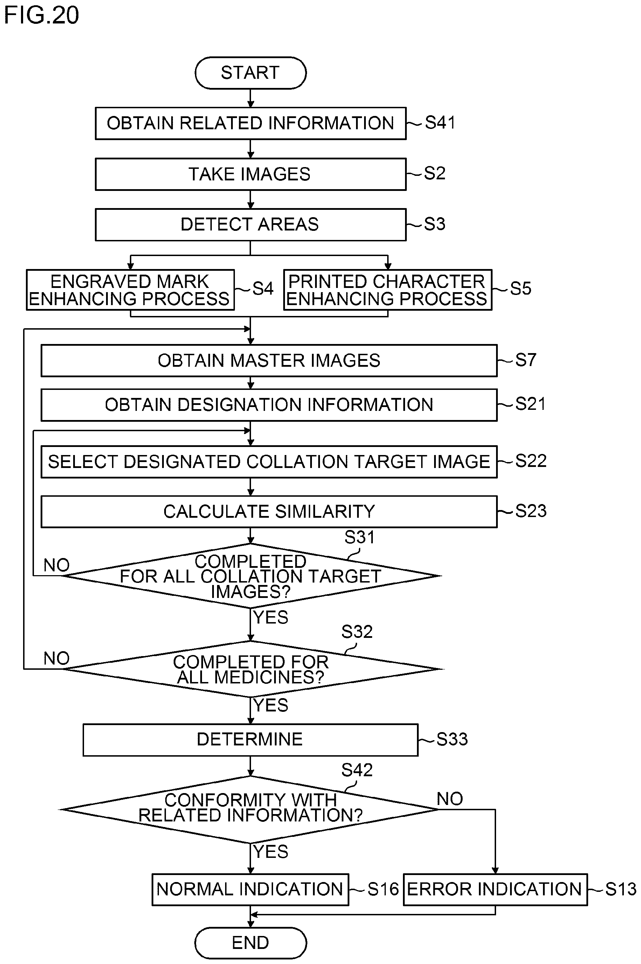

Figure 20 is a flowchart showing processes of a medicine inspection support method by the medicine inspection support device 4 according to a sixth embodiment. Note that the parts common to those of the flowchart shown in Figure 19 are assigned the same numerals or characters; their detailed description is omitted.

Steps S41 to S5 are analogous to those of the fifth embodiment. That is, the related information is obtained. The medicines placed on the Petri dish SC are imaged to obtain a plurality of taken images. Medicine areas where the medicines are imaged are detected from the taken images. The first enhanced images where the engraved mark portions of the medicines are enhanced, and the second enhanced images where the printed character portions of the medicines are enhanced are generated.

In step S7, a pair of master images is obtained. In step S21, the designation information associated with the master image is obtained. Furthermore, in step S22, the images designated by the designation information are selected from either the first enhanced images or the second enhanced images of the medicine to be collated.

In step S23, the similarity between the pair of master images obtained in step S7 and the pair of collation target images selected in step S22 is calculated. The similarity described here is calculated such that the greater the degree of the similarity property is, the higher the value of the similarity is.

Next, in step S31, the processing unit 10 determines whether or not calculation of the similarity is completed for the combinations between all the collation target images and the master images obtained in step S7. If there is any collation target image whose similarity has not been calculated yet, the processing returns to step S22, a new pair of collation target images is selected and analogous processes are repeated. Accordingly, for the master images obtained in step S7, the similarities of the combinations with all the collation target images can be calculated. If the similarity calculation for the combinations with all the collation target images has been completed, the processing transitions to step S32.

Subsequently, in step S32, the processing unit 10 determines whether similarity calculation for all the medicines whose related information have been obtained is completed or not. If there is any medicine whose similarity calculation has not been completed yet, the processing returns to step S7, a new pair of master images is obtained and analogous processes are repeated. Accordingly, for every master image, the similarities of the combinations with all the collation target images obtained in step S22 can be calculated. If the similarity calculation for all the master images has been completed, the processing transitions to step S33.

In step S33, the determining unit 24 determines whether or not the medicines whose related information have been obtained are the same as the imaged medicines based on the similarities of all the combinations between the master images and the collation target images calculated in step S23.

In step S42, based on the determination result in step S33, it is determined whether the medicines placed on the Petri dish SC conform to the obtained related information or not. If it is determined that the medicines do not conform to the related information, the processing transitions to step S13. If it is determined the medicines conform to the related information, the processing transitions to step S16. In step S13, the display unit 50 displays the error indication on the monitor 52, and the processing of this flowchart is finished.

In step S16, the display unit 50 displays the normal indication on the monitor 52, and the processing of this flowchart is finished.

As described above, even in a case where the present invention is applied to a device that obtains related information on medicines and performs medicine differentiation, correct collation can be performed by determining combinations between the medicines to be dispensed and the dispensed medicines, using relative relationship of similarities.

### <Seventh embodiment>

A medicine inspection support device according to a seventh embodiment inspects medicines which are dispensed based on prescription data and packaged in divided packages of a strip medicine package, and displays a list table of medicines.

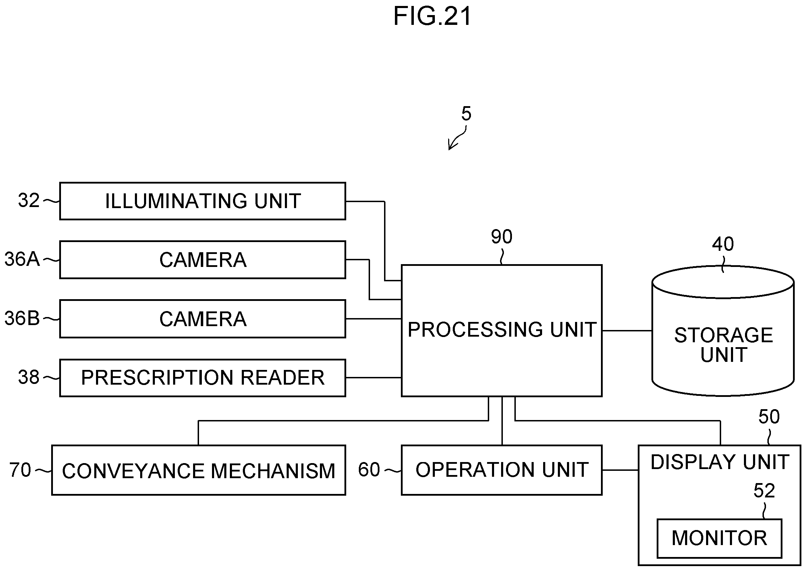

Figure 21 shows the configuration of a medicine inspection support device 5 according to the seventh embodiment. Note that the parts common to those of the medicine inspection support device 1 shown in Figure 1 are assigned the same numerals or characters; their detailed description is omitted. The medicine inspection support device 5 includes a processing unit 90.

### [Configuration of processing unit]

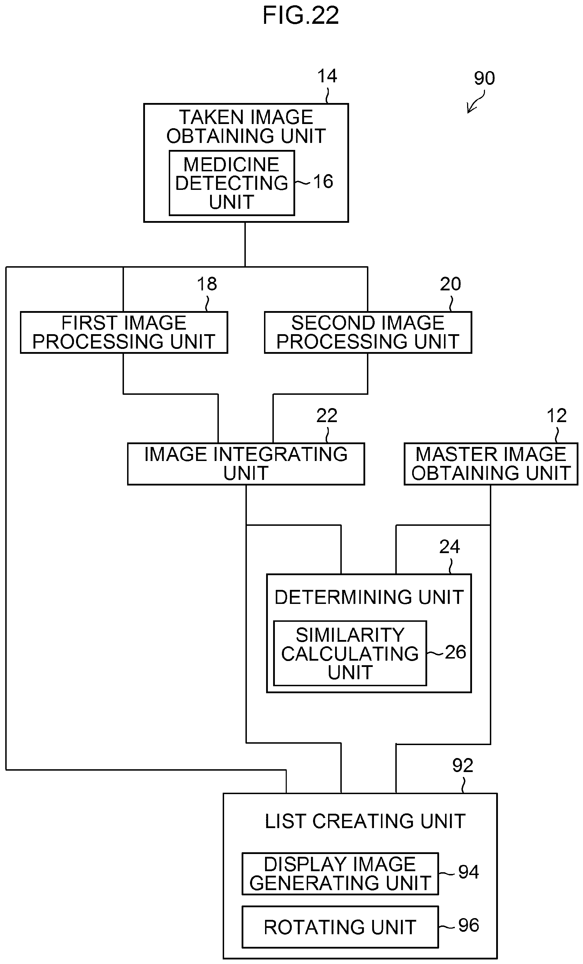

Figure 22 shows the internal configuration of the processing unit 90. Note that the parts common to those of the processing unit 10 shown in Figure 4 are assigned the same numerals or characters; their detailed description is omitted.

The determining unit 24 corresponds to a medicine discriminating unit. The determining unit 24 collates medicine master images from the master image obtaining unit 12 with taken images from the taken image obtaining unit 14, and determines medicines with which the medicines present in the taken images coincide.

The processing unit 90 includes a list creating unit 92. The list creating unit 92 creates a list table for medicines (example of medicines to be inspected) to be packaged in divided packages in a divided manner. The list creating unit 92 obtains images of the medicines packaged in the divided package, from the taken image obtaining unit 14. Here, images (medicine area images) of medicine areas that are areas where the medicines are imaged and detected by the medicine detecting unit 16, are obtained. The list creating unit 92 obtains integrated images of the medicines packaged in the divided package, from the image integrating unit 22. Furthermore, the list creating unit 92 obtains, from the master image obtaining unit 12, the master images of medicines which may be dispensed based on the prescription data.

The list creating unit 92 includes a display image generating unit 94, and a rotating unit 96.

The display image generating unit 94 generates difference images obtained by subtracting, from the integrated images, the medicine area images corresponding to the integrated images. The difference image is a display image where the engraved mark portion or the printed character portion of a medicine is enhanced.

The rotating unit 96 rotates the display images, thereby aligning the engraved mark portions or the printed character portions of the display images and those of the master images corresponding to the display images in the same orientation.

### [Processes of medicine inspection support method]

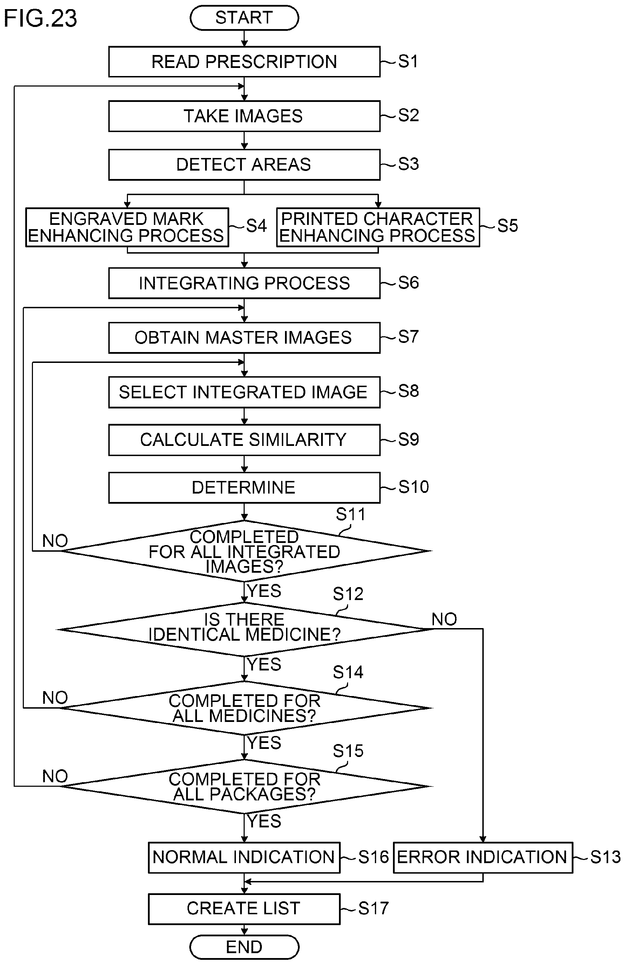

A medicine inspection support method by the medicine inspection support device 5 is described. Figure 23 is a flowchart showing the processes of the medicine inspection support method. The processes in steps S1 to S16 are analogous to those of the flowchart shown in Figure 5.

After the error indication in step S13 or the normal indication in step S16 is performed, the processing transitions to step S17. In step S17, the list creating unit 92 creates a list table, and displays the created list table on the monitor 52 of the display unit 50.

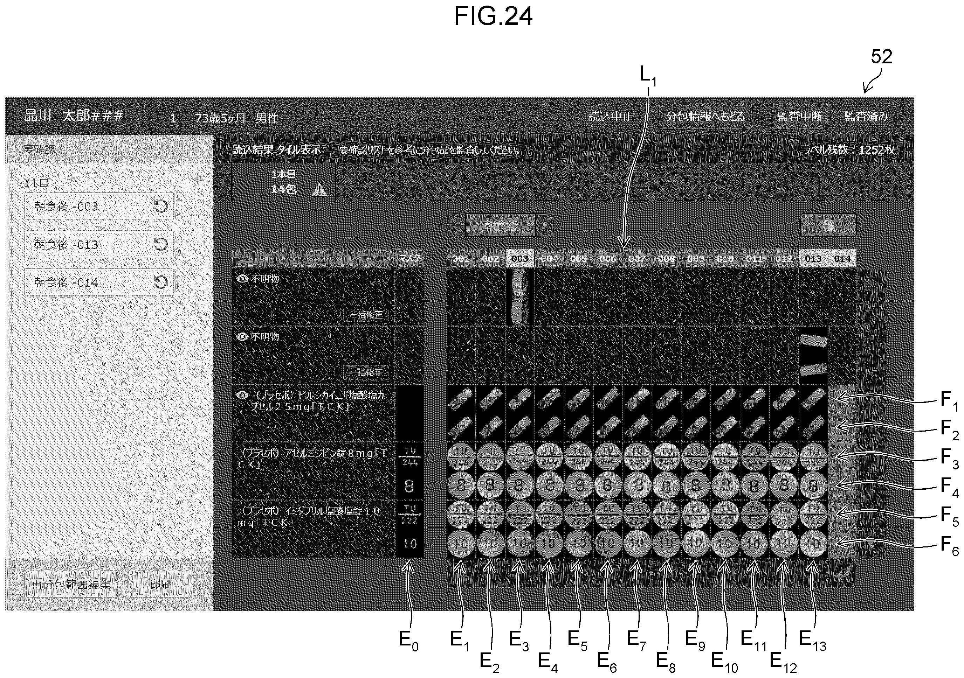

Figure 24 shows the list table L₁ displayed on the monitor 52. Here, an example is shown where a medicine A, a medicine B and a medicine C are packaged in each of thirteen divided packages. The list table L₁ includes a column E₀ displaying the display images of the master images of the medicines to be dispensed, and columns E₁ to E₁₃ displaying the display images of the medicines packaged in each divided package.

Each of columns E₀ to E₁₃ is divided into a row F₁ displaying the display image on the front side of the medicine A, a row F₂ displaying the display image on the rear side of the medicine A, a row F₃ displaying the display image on the front side of the medicine B, a row F₄ displaying the display image on the rear side of the medicine B, a row F₅ displaying the display image on the front side of the medicine C, and a row F₆ displaying the display image on the rear side of the medicine C.

On each of the rows F₁ to F₆ on each of the columns E₀ to E₁₃, the display image corresponding to the position is displayed. The display image of the medicine and the display image of the master image, which are displayed here, are aligned by the rotating unit 96 such that the engraved mark portions or the printed character portions are oriented in the same direction.

As shown in Figure 24, the list table is displayed where the master image of the medicine to be dispensed and the medicine area image of each medicine are positionally aligned and the engraved mark portion and the printed character portion are enhanced. Accordingly, display easily recognizable by the user can be achieved. Note that the engraved mark portion and the printed character portion are enhanced here. Alternatively, a list table where one of the engraved mark portion and the printed character portion is enhanced may be displayed. Both the cases are encompassed by the concept of the list table where the engraved mark portion or the printed character portion is enhanced.

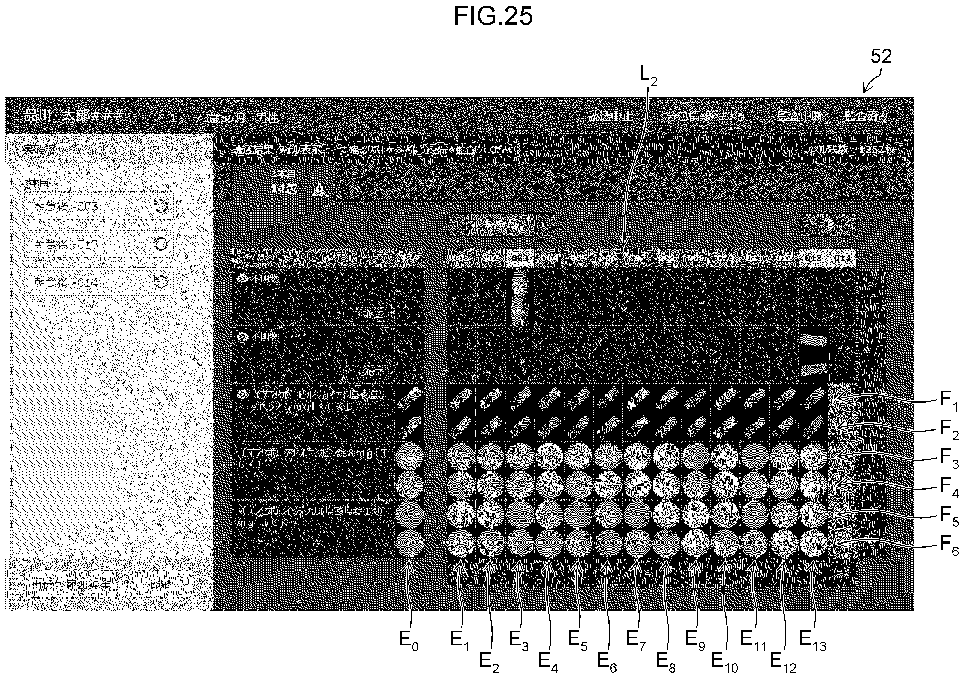

Figure 25 shows a list table L₂ of a comparative example. The list table L₂ shown in Figure 25 displays the master images and the medicine area images without enhancement of the engraved mark portion and the printed character portion. The medicine area images are viewed in a different manner depending on the positional relationship between the light source and the engraved mark portion. Here, the list table L₂ has a poor viewability.

In this embodiment, the difference image obtained by subtracting the medicine area image from the integrated image is used as the display image in which the engraved mark portion or the printed character portion of the medicine is enhanced. However, the display image displayed in the list table is not limited to the difference image, as long as the method can improve user's viewability.

In this embodiment, the example where medicines packaged in divided packages are inspected is described. Alternatively, the medicine inspection support device 5 can inspect medicines placed on a Petri dish or medicines directly placed on a stage. However, inspection of the medicines packaged in the divided packages is useful and preferable in view of operability.

Note that the medicine inspection includes audit and differentiation (identification). In this embodiment, the example of supporting audit of medicines dispensed based on the prescription data is described. Alternatively, the medicine inspection support device 5 is applicable to supporting medicine differentiation. The medicine differentiation is to identify a medicine from an appearance of the medicine. The medicine differentiation can determine medicines carried by a hospitalized patient, for example.

### <Other>

The image processing method described above can be configured as a program of causing a computer to achieve the master image obtaining function, the taken image obtaining function, the first image processing function, the second image processing function, the image integrating function, the designation information obtaining function, and the determining function, and configured as a non-transitory recording medium, such as a CD-ROM (Compact Disk-Read Only Memory), storing the program.

In the thus described embodiments, for example, the hardware structure of the processing unit that executes various kinds of processes, such as of the processing unit 10 and the processing unit 11, is any of various processors as described below. The various processors include: a CPU (Central Processing Unit), which is a general-purpose processor executing software (program) to function as various processing units; a GPU (Graphics Processing Unit) which is a processor specifically dedicated to image processing; a programmable logic device (PLD), such as an FPGA (Field Programmable Gate Array), which is a processor whose circuit configuration can be changed after production; and dedicated circuitry, such as an ASIC (Application Specific Integrated Circuit), which is a processor having a circuit configuration designed in a dedicated manner to execute a specific process.

One processing unit may include one among these various processors, or include the same kind or different kinds of two or more processors (e.g., multiple FPGAs, a combination of a CPU and an FPGA, or a combination of a CPU and a GPU). Alternatively, multiple processing units may be made up of a single processor. Examples where multiple processing units are made up of a single processor include, firstly, a mode where as typified by a computer, such as a server and a client, a combination of one or more CPUs and software constitute a single processor, and the processor functions as multiple processing units. Secondly, as typified by a system on chip (SoC), the examples include a mode of using a processor where the function of the entire system including multiple processing units is achieved by a single IC (Integrated Circuit) chip. As described above, various processing units are configured using one or more various processors as a hardware configuration.

Furthermore, more specifically, each of the hardware structures of these various processors is circuitry including combined circuit elements, such as semiconductor elements.

The technical scope of the present invention is not limited to the scope described in the above embodiments. The configurations and the like in the respective embodiments can be appropriately combined between the embodiments in a range without departing from the spirit of the present invention.

### Reference Signs List

1 Medicine inspection support device
2 Medicine inspection support device
3 Medicine inspection support device
4 Medicine inspection support device
5 Medicine inspection support device
10 Processing unit
11 Processing unit
12 Master image obtaining unit
14 Taken image obtaining unit
16 Medicine detecting unit
18 First image processing unit
20 Second image processing unit
22 Image integrating unit
24 Determining unit
26 Similarity calculating unit
28 Designation information obtaining unit
32 Illuminating unit
34 Light sources
34E Light sources
34N Light sources
34S Light sources
34W Light sources
36A Camera
36B Camera
38 Prescription reader
40 Storage unit
50 Display unit
52 Monitor
60 Operation unit
70 Conveyance mechanism
80 Related information obtaining unit
90 Processing unit
92 List creating unit
94 Display image generating unit
96 Rotating unit
A₁ to A₄₄ Medicine area
B₁ to B₄₄ First enhanced image
C₁ to C₄₄ Second enhanced image
D₁ to D₄₄ Integrated image
E₀ to E₁₃ Column
F₁ to F₆ Row
L₁ List table
L₂ List table
PA Imaging optical axis
PB Strip medicine package
SC Petri dish
TP Divided package
S1 to S35 Steps of medicine inspection support method in medicine inspection support device

## Claims

1. An image processing device, comprising:
a master image obtaining unit configured to obtain a master image of a first medicine;
a taken image obtaining unit configured to obtain a plurality of taken images of a second medicine, with light emitting directions to a surface of the second medicine different from each other;
a first image processing unit configured to perform a process of enhancing an engraved mark portion of the second medicine based on at least one taken image among the taken images, and generate a first enhanced image;
a second image processing unit configured to perform a process of enhancing a printed character portion of the second medicine based on at least one taken image among the taken images, and generate a second enhanced image;
a designation information obtaining unit configured to obtain designation information designating one of the first enhanced image and the second enhanced image, as an image to be used for collation; and
a determining unit configured to collate the image designated by the designation information with the master image, and determine whether the first medicine is identical to the second medicine or not.

2. The image processing device according to claim 1, wherein the master image and the designation information are stored in association with each other.

3. The image processing device according to claim 1 or 2, further comprising
a medicine detecting unit configured to detect areas where the second medicine are imaged from the taken images,
wherein the first image processing unit and the second image processing unit perform the processes for the detected areas.

4. The image processing device according to any one of claims 1 to 3,
wherein the taken image obtaining unit obtains three or more taken images, and
the first image processing unit generates the first enhanced image based on the three or more taken images.

5. The image processing device according to claim 4, wherein the first image processing unit obtains three-dimensional information on the surface of the second medicine by photometric stereo, and generates the first enhanced image.

6. The image processing device according to any one of claims 1 to 5, wherein the second image processing unit performs at least one of a smoothing process, a sharpening process and an edge detecting process, and generates the second enhanced image.

7. The image processing device according to any one of claims 1 to 6, further comprising
a similarity calculating unit configured to calculate a similarity indicating a degree at which the image designated by the designation information is similar to the master image,
wherein the determining unit determines whether the first medicine is identical to the second medicine or not, based on the similarity.

8. The image processing device according to claim 7,
wherein the similarity calculating unit calculates similarities between a plurality of images designated by the designation information and a plurality of master images, and
the determining unit determines that the first medicine is identical to the second medicine, for a combination between an image designated by the designation information and a master image, with a highest similarity.

9. The image processing device according to any one of claims 1 to 8, wherein the master image obtaining unit obtains the master image of the first medicine based on prescription data.

10. A medicine inspection support device, comprising:
a master image obtaining unit configured to obtain a master image of a first medicine;
an illuminating unit including a plurality of light sources and configured to irradiate a surface of a second medicine with light in a plurality of illumination directions different from each other;
an imaging unit configured to image the second medicine;
an imaging control unit configured to control the illuminating unit and the imaging unit, and obtain a plurality of taken images of the second medicine with light illumination directions to the second medicine different from each other;
a first image processing unit configured to perform a process of enhancing an engraved mark portion of the second medicine based on at least one taken image among the taken images, and generate a first enhanced image;
a second image processing unit configured to perform a process of enhancing a printed character portion of the second medicine based on at least one taken image among the taken images, and generate a second enhanced image;
a designation information obtaining unit configured to obtain designation information designating one of the first enhanced image and the second enhanced image, as an image to be used for collation; and
a determining unit configured to collate the image designated by the designation information with the master image, and determine whether the first medicine is identical to the second medicine or not.

11. The medicine inspection support device according to claim 10, further comprising
a master image storage unit configured to store the master image of the first medicine,
wherein the designation information is stored in the master image storage unit in association with the master image.

12. The medicine inspection support device according to claim 10 or 11, wherein the imaging control unit obtains the taken images of the second medicine irradiated with light by two or more light sources among the light sources of the illuminating unit.

13. The medicine inspection support device according to any one of claims 10 to 12,
wherein the illuminating unit irradiates a front side and a rear side of the second medicine with light, and
the imaging unit images the front side and the rear side of the second medicine.

14. The medicine inspection support device according to any one of claims 10 to 13,
wherein the illuminating unit comprises a first light source configured to emit light in a first direction, a second light source configured to emit light in a second direction, a third light source configured to emit light in a third direction, and a fourth light source configured to emit light in a fourth direction, and
the second direction is a direction opposite to the first direction in plan view of the surface, the third direction is a direction orthogonal to the first direction in plan view of the surface, and the fourth direction is a direction opposite to the third direction in plan view of the surface.

15. An image processing method, comprising:
a master image obtaining step of obtaining a master image of a first medicine;
a taken image obtaining step of obtaining a plurality of taken images of a second medicine, with light emitting directions to a surface of the second medicine different from each other;
a first image processing step of performing a process of enhancing an engraved mark portion of the second medicine based on at least one taken image among the taken images, and generating a first enhanced image;
a second image processing step of performing a process of enhancing a printed character portion of the second medicine based on at least one taken image among the taken images, and generating a second enhanced image;
a designation information obtaining step of obtaining designation information designating one of the first enhanced image and the second enhanced image, as an image to be used for collation; and
a determining step of collating the image designated by the designation information with the master image, and determining whether the first medicine is identical to the second medicine or not.

16. A program causing a computer to execute:
a master image obtaining function of obtaining a master image of a first medicine;
a taken image obtaining function of obtaining a plurality of taken images of a second medicine, with light emitting directions to a surface of the second medicine different from each other;
a first image processing function of performing a process of enhancing an engraved mark portion of the second medicine based on at least one taken image among the taken images, and generating a first enhanced image;
a second image processing function of performing a process of enhancing a printed character portion of the second medicine based on at least one taken image among the taken images, and generating a second enhanced image;
a designation information obtaining function of obtaining designation information designating one of the first enhanced image and the second enhanced image, as an image to be used for collation; and
a determining function of collating the image designated by the designation information with the master image, and determining whether the first medicine is identical to the second medicine or not.

17. A non-transitory and computer-readable recording medium wherein when an instruction stored in the recording medium is read by a computer, the instruction causes the computer to execute:
a master image obtaining function of obtaining a master image of a first medicine;
a taken image obtaining function of obtaining a plurality of taken images of a second medicine with light emitting directions to a surface of the second medicine different from each other;
a first image processing function of performing a process of enhancing an engraved mark portion of the second medicine based on at least one taken image among the taken images, and generating a first enhanced image;
a second image processing function of performing a process of enhancing a printed character portion of the second medicine based on at least one taken image among the taken images, and generating a second enhanced image;
a designation information obtaining function of obtaining designation information designating one of the first enhanced image and the second enhanced image, as an image to be used for collation; and
a determining function of collating the image designated by the designation information with the master image, and determining whether the first medicine is identical to the second medicine or not.

18. A medicine inspection support device inspecting a medicine, comprising:
a medicine discriminating unit configured to collate a medicine master image from a medicine database including medicine master images with a taken image obtained by imaging a medicine to be inspected, and determine which medicine the medicine present in the taken image is; and
a list creating unit configured to create a list table of medicines to be inspected, the list table displaying the medicine master image and a medicine area image determined to correspond to each medicine in the taken image, with positions of the medicine master image and the medicine area image being aligned to each other, and an engraved mark portion or a printed character portion being enhanced.
